(19) Europäisches Patentamt
European Patent Office
Office européen des brevets

(11) EP 3 382 033 B1

(12) **EUROPEAN PATENT SPECIFICATION**

(45) Date of publication and mention of the grant of the patent:
**05.08.2020 Bulletin 2020/32**

(51) Int Cl.:
***C12Q 1/6881*** *(2018.01)* ***C12Q 1/6883*** *(2018.01)*
***C12Q 1/6886*** *(2018.01)*

(21) Application number: **17163798.6**

(22) Date of filing: **30.03.2017**

(54) **METHOD FOR DETERMINING BLOOD COUNTS BASED ON DNA METHYLATION**

VERFAHREN ZUR BESTIMMUNG VON BLUTKÖRPERCHENZAHLEN AUF DER BASIS DER DNS METHYLIERUNG

MÉTHODE POUR DÉTERMINER LE NOMBRE DES CELLULES SANGUINES BASÉE SUR LA MÉTHYLATION DE L'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.10.2018 Bulletin 2018/40**

(73) Proprietor: **Rheinisch-Westfälische Technische Hochschule (RWTH) Aachen**
**52056 Aachen (DE)**

(72) Inventors:
- **WAGNER, Wolfgang**
  **52078 Aachen (DE)**
- **FROBEL, Joana**
  **52066 Aachen (DE)**

(74) Representative: **Remus, Alvaro Johannes**
**BPSH Schrooten Haber Remus**
**Patent- und Rechtsanwaltspartnerschaft mbB**
**Grafenberger Allee 277-287, Eingang A**
**40237 Düsseldorf (DE)**

(56) References cited:
**EP-A1- 2 199 411**
**EP-A1- 2 248 913**
**WO-A1-2015/129916**
**WO-A1-2016/041010**
**WO-A1-2016/109712**
**WO-A2-03/045230**
**WO-A2-2012/088298**
**WO-A2-2012/162660**
**WO-A2-2013/033627**
**WO-A2-2014/170497**
**DE-A1- 10 044 543**
**US-A1- 2007 150 978**
**US-A1- 2015 004 602**

- **SIMONE BORK ET AL: "DNA methylation pattern changes upon long-term culture and aging of human mesenchymal stromal cells", AGING CELL, BLACKWELL PUBLISHING, GB, vol. 9, no. 1, 1 February 2010 (2010-02-01), pages 54-63, XP002663876, ISSN: 1474-9718, DOI: 10.1111/J.1474-9726.2009.00535.X [retrieved on 2009-11-06]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 382 033 B1

## Description

### Background of the invention

The invention relates to a method for determining at least a part of the cellular composition of a blood sample, with which the relative distribution of at least some blood cell types in the sample can be predicted. The invention further relates to the use of an artificial nucleic acid molecule for determining at least a part of the cellular composition of blood.

### Prior art

Analysis of the composition of different types of white blood cells is one of the most frequently used diagnostic tests. Leukocyte differential counts (LDCs) can be determined by microscopic evaluation and manual counting. However, since the advent of automated cell counters, LDCs are particularly analyzed by flow cytometric technologies. Such automated analyzers sense electrical impedance, optical light scattering properties, or fluorescence signal intensities. Fluorescent staining of specific epitopes is the gold standard for definition of lymphocyte subsets. However, immunophenotypic analysis is relatively labor-intensive and difficult to standardize. Furthermore, all of the above mentioned methods are only applicable to fresh blood samples - it is not possible to freeze samples for shipment or later analysis. Recently, gene expression profiles as well as epigenetic profiles (Accomando et al., 2014; Houseman et al., 2012) have been used to deconvolute the cellular composition in whole blood. Such alternative approaches might overcome some of the limitations of the well-established state of the art procedures for LDCs.

DNA-methylation (DNAm) represents the best understood epigenetic modification. Methyl groups can be added to the $5^{th}$ carbon atom of cytosines, predominantly in a cytosine-guanine (CpG) dinucleotide context. Many studies demonstrated that genome-wide DNAm profiles can be used to estimate LDCs (Adalsteinsson et al., 2012; Houseman et al., 2015; Jaffe and Irizarry, 2014; McGregor et al., 2016; Waite et al., 2016). In fact, DNAm patterns have many advantages in comparison to immunophenotypic analysis and RNA profiling: i) DNAm is directly linked to the process of cellular differentiation; ii) it facilitates absolute quantification at single base resolution (ranging from 0 to 100% DNAm); iii) every leucocyte cell has only two copies of DNA and hence the results can be easily extrapolated to the cellular composition (whereas RNA can be highly overexpressed in small subsets); and iv) DNA is relatively stable: it can be isolated from lysed or frozen cells and shipped at room temperature for further analysis. Deconvolution algorithms that are based on a-priori reference datasets of cell type-specific DNAm patterns can be used to estimate the cellular composition in heterogeneous tissues (Teschendorff et al., 2017). In principle, it is also possible to train reference-free algorithms, but since the composition of blood is relatively well known, reference-based algorithms may be advantageous (Zheng et al., 2017). So far, the available algorithms for LDCs are based on a multitude of CpGs of 450k Illumina BeadChip microarray data. It should be possible to translate the established algorithms to the newer EPIC array or to whole genome bisulfite sequencing (WGBS) and reduced representation bisulfite sequencing (RRBS) data (BLUEPRINT consortium, 2016). However, all of these profiling procedures are hardly applicable in daily clinical routine.

WO 2014/170497 A2 discloses an epigenetic haemogram that identifies the quantitative, comprehensive picture of cellular composition in a biological sample, wherein a normalization standard is used. The normalization standard is a nucleic acid molecule comprising at least one marker-region being specific for each of the blood cells to be detected, and at least one control-region being cell-unspecific, wherein said regions are present in the same number of copies on said molecule and/or a natural blood cell sample of known composition. The biological sample is derived from mammalian body fluid, including peripheral, capillary or venous blood samples or subfractions thereof, such as peripheral blood mononuclear cells or peripheral blood monocytes, or a tissue sample, organ sample, or from frozen, dried, embedded, stored or fresh body fluids or tissue samples.

EP 2 199 411 A1 relates to a method for identifying CD3CD4 positive T lymphocytes of a mammal, wherein said method comprises analyzing the methylation status of at least one CpG position in the CD3a/b/c/d/g genes, in particular their "upstream" regulatory regions, and in particular the promoter and other conserved regions of the gene cd3, wherein a demethylation of at least one CpG in the analyzed sample to at least 90% is indicative for memory and naive CD4 or/and memory and/or naive T lymphocytes. Accordingly, the method is directed to the use of DNA-methylation analysis of the genes CD3a/b/c/d for the detection and quality assurance and control of T lymphocytes. To this end, the methylation status of at least one CpG position in the gene CD3 is analyzed, allowing for a precise analysis even from sub-optimal quality samples, such as non-freshly obtained blood or serum samples.

WO 2012/162660 A2 discloses methods using DNA methylation arrays for identifying a cell or mixture of cells and for quantification of alterations in distribution of cells in blood or in tissues, and for diagnosing, prognosing and treating disease conditions, particularly cancer. In these methods, a CD3Z positive T lymphocyte cell number is measured in a sample from the subject by analyzing methylation in the sample of at least one CpG dinucleotide (CpG) in gene CD3Z or in an orthologous or a paralogous gene thereof, such that an amount of a demethylated C of the at least one CpG in the sample is a measure of CD3+ T lymphocyte cell number. The amount of the demethylated C in the sample from the subject is compared with that in positive control samples from patients with the disease condition, and with that in negative control samples from healthy subjects, wherein the disease condition is selected from an autoimmune disease, an allergy, a transplant rejection, obesity, an inherited disease, immunosuppression, and a cancer.

EP 2 248 913 A1 relates to a method for identifying natural killer cells of a mammal, which often express the surface proteins CD16 and/or CD56, wherein the methylation status of at least one CpG position in the CX3CR1 and/or FGR and/or NKG7 and/or GNLY genes is analyzed, and wherein a demethylation of at least one CpG in the analyzed sample to at least 70% is indicative for CD56 expressing NK cells, which might also be CD8+ or CD8-, CD56 dim or bright, CD16+ or CD16- NK cells. The methods of the present invention are useful for the detection and quality assurance and control of NK cells. The method allows for analysis even from sub-optimal quality samples, such as non-freshly obtained blood, tissue or serum samples.

US 2015/0004602 A1 concerns a method for identifying CD3CD4 positive T lymphocytes of a mammal, comprising analyzing the bisulfite convertibility of at least one CpG position in the CD3+CD4+ T helper cell specific non-methylated bisulfite convertible region, wherein a bisulfite convertibility of at least one CpG position to at least 90% in said sample is indicative for a CD4+ T-lymphocyte cell, in particular a CD3+ CD4+ T-lymphocyte cell. The method further relates to analyzing the bisulfite convertibility of at least one CpG position in the genes FLJ00060, FLJ38379, PPP6C, CD226, ZBTB7B and TNFAIP8 that are capable of positively identifying CD4 expressing cells in whole blood and segregate between CD4 and CD8 positive CD3 positive cells.

## Summary of the invention

It is the object of the invention to provide a method for determining at least a part of the cellular composition of a blood sample, with which relative cell counts can be determined based on DNAm patterns, and which ensure similar precision as conventional methods.

The object is achieved by providing a method for determining at least a part of the cellular composition of blood, comprising:

- Isolating genomic DNA from at least one blood sample so as to obtain at least one nucleic acid preparation of said blood sample;
- Identifying DNA methylation levels of at least two different specific region of said nucleic acid preparation, each specific region comprising at least one CpG-dinucleotide, wherein each of said two different specific regions comprises at least one nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13; and
- Determining the proportions of at least two different blood cell types based on the identified methylation levels so as to predict the relative distribution of said at least two different blood cell types in the blood sample, wherein the methylation level of at least one CpG-dinucleotide selected from the group consisting of nucleotides no. 6 and 7, 38 and 39, 49 and 50, 51 and 52, 61 and 62, 80 and 81, 89 and 90, and 112 and 113 of SEQ ID NO: 1 is indicative of the percentage of granulocytes, the methylation level of at least one CpG-dinucleotide selected from the group consisting of nucleotides no. 40 and 41, 45 and 46, 53 and 54, and 61 and 62 of SEQ ID NO: 5 is indicative of the percentage of monocytes, the methylation level of nucleotides no. 61 and 62 of SEQ ID NO: 6 is indicative of the percentage of lymphocytes, the methylation level of at least one CpG-dinucleotide selected from the group consisting of nucleotides no. 15 and 16, 26 and 27, 32 and 33, and 61 and 62 of SEQ ID NO: 2 is indicative of the percentage of neutrophil granulocytes, the methylation level of at least one CpG-dinucleotide selected from the group consisting of nucleotides no. 6 and 7, 21 and 22, 32 and 33, 34 and 35, 54 and 55, 61 and 62, 63 and 64, 81 and 82, 93 and 94, 101 and 102, and 121 and 122 of SEQ ID NO: 3, or selected from the group consisting of nucleotides no. 1 and 2, 12 and 13, 14 and 15, 34 and 35, 41 and 42, 43 and 44, 61 and 62, 73 and 74, 81 and 82, and 101 and 102 of SEQ ID NO: 4 is indicative of the percentage of eosinophil granulocytes, the methylation level of at least one CpG-dinucleotide selected from the group consisting of nucleotides no. 5 and 6, 36 and 37, 42 and 43, 53 and 54, 58 and 59, 61 and 62, and 63 and 64 of SEQ ID NO: 7 is indicative of the percentage of B-cells, the methylation level of at least one CpG-dinucleotide selected from the group consisting of nucleotides no. 25 and 26, 61 and 62, 64 and 65, 81 and 82, and 104 and 105 of SEQ ID NO: 8, or selected from the group consisting of nucleotides no. 22 and 23, 58 and 59, 61 and 62, 77 and 78, and 100 and 101 of SEQ ID NO: 9, or selected from the group consisting of nucleotides no. 6 and 7, 42 and 43, 45 and 46, 61 and 62, 84 and 85, and 115 and 116 of SEQ ID NO: 10 is indicative of the percentage of NK-cells, the methylation level of at least one CpG-dinucleotide selected from the group consisting of nucleotides no. 17 and 18, 26 and 27, 34 and 35, 46 and 47, 61 and 62, 92 and 93, 96 and 97, and 108 and 109 of SEQ ID NO: 11 is indicative of the percentage of T-cells, the methylation level of nucleotides no. 20 and 21, and/or 61 and 62 of SEQ ID NO: 12 is indicative of the percentage of CD4+ T-cells, and the methylation level of nucleotides no. 61 and 62 of SEQ ID NO: 13 is indicative of the percentage of CD8+ T-cells.

Surprisingly, even site-specific analysis of DNA methylation (DNAm) at individual CpG sites can be utilized to determine leukocyte differential counts (LDCs). The method according to the invention facilitates robust estimation of the cellular composition in blood to overcome limitations of conventional blood counts. The method according to the invention is not restricted to the analysis of fresh blood samples since it is also possible to freeze the samples, e.g., for shipment or later analysis. According to the invention even DNAm levels at individual CG-dinucleotides (CpGs) reflect fractions of granulocytes (including neutrophil and eosinophil granulocytes), CD4+ T-cells, CD8+ T-cells, B-cells, NK-cells, or monocytes. The method facilitates site-specific analysis, e.g. by pyrosequencing or MassARRAY technology, and reaches similar precision as conventional LDC methods. The method according to the invention (in the following occasionally referred to as "Epi-Blood-Count") is applicable to frozen samples, upon long-term storage, and to very small volumes of blood, and enables more standardized and cost-effective analysis of blood counts in clinical application.

SEQ ID NO: 1

Part of the nucleotide sequence (DNA, R strand) of the *WDR20* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

AGGTG**CG**GTGGGGGAGGGGGATTGGGTCAGAAAGGCA**CG**TGACTCCTC**CG**
**CG**CTTTGTTT[**CG**]TAAAGCCTGAGACTCCC**CG**AGTCTTC**CG**ACTTCTCCCTGA
CATGGCCCT**CG**TTTGGGCTT (SEQ ID NO: 1)

SEQ ID NO: 2

Part of the nucleotide sequence (DNA, F strand) of the *PCYOX1* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

GCTGCCTCTGCTTC**CG**TTTCCTCCA**CG**AGGG**CG**AAGGTATACCCAGAACAAA
TTATGCCA[**CG**]AAGATGCATGTCTGACATCTTTGTTTTTAAATTGAATTCAGTT
GGTACTCTGGCTTTTTT (SEQ ID NO: 2)

SEQ ID NO: 3

Part of the nucleotide sequence (DNA, F strand) of the *RPS6KA2* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

GAGCC**CG**CAACCCCTGACAA**CG**GGAGGAAGA**CGCG**CCACAGGGACTGGGA
CAG**CG**GCCAC[**CG**]**CG**GTGACACCTACAGCCA**CG**CAAGCACCTG**CG**TAAACA
**CG**TGCTACAGTCACATGTTA**CG** (SEQ ID NO: 3)

SEQ ID NO: 4

Part of the nucleotide sequence (DNA, F strand) of the *RPS6KA2* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

**CG**GGAGGAAGA**CGCG**CCACAGGGACTGGGACAG**CG**GCCAC**CGCG**GTGACA
CCTACAGCCA[**CG**]CAAGCACCTG**CG**TAAACA**CG**TGCTACAGTCACATGTTA**C**
**G**TATTTCACACATTTACACAC (SEQ ID NO: 4)

SEQ ID NO: 5

Part of the nucleotide sequence (DNA, F strand) of the *CENPA* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

ACCTTGGCCTCCCAGAGTGCTGGGATTACAGGCCTGAGC**<u>CG</u>**CTG**<u>CG</u>**CCTGGC**<u>CG</u>**TATTTA[**CG**]ATCTTAAGAGAAAGGGCAGGAGTGTTTCCTTGACCCCACATTCTCACTTCCCTATACCTC (SEQ ID NO: 5)

SEQ ID NO: 6

Part of the nucleotide sequence (DNA, R strand) of the *FYN* gene. CpG-dinucleotide site in close proximity to the CpG site presented by the Illumina Infinium HumanMethylation450 BeadChip (Illumina, San Diego, USA), which is printed in bold letters and put in squared brackets, is underlined and printed in bold letters:

TAAGTACTTTGCATAATGCAAAGCTTGTGCCTCTAGCTAATTTGCAGGCAGATGACACAG[**CG**]TGAGAAGCCTCTGATTTAGTAGAATAAGAACTATTTGTAACCAAACAGCCTCCCTCTGTT (SEQ ID NO: 6)

SEQ ID NO: 7

Part of the nucleotide sequence (DNA, R strand) of the *WIPI2* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

CTGC**<u>CG</u>**TTGGGTTTTTCATATCTGTACATAATGC**<u>CG</u>**AGTG**<u>CG</u>**TAAGGAAAC**<u>CG</u>**TGG**<u>CG</u>**T[**CG**]**<u>CG</u>**CACAGTGGGTCTGCTTGTCAAGGCCAGTTCTGCAGTGACAGGCCCAGGGGCTGCCCAC (SEQ ID NO: 7)

SEQ ID NO: 8

Part of the nucleotide sequence (DNA, F strand) of the *SLC15A4* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

CATCAGGAGGCTTGGTGATGAAAA**<u>CG</u>**CTCTGGCCACAGAGGAAGACCACAAAAGCAAGGC[**CG**]A**<u>CG</u>**CAGACAGTGGGGAT**<u>CG</u>**CATAACCAGTGACAAAGCTGA**CG**TTCTGCTGAATATAGGCA (SEQ ID NO: 8)

SEQ ID NO: 9

Part of the nucleotide sequence (DNA, F strand) of the *SLC15A4* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

CAGGAGGCTTGGTGATGAAAA**CG**CTCTGGCCACAGAGGAAGACCACAAAAG CAAGGC**CG**A[**CG**]CAGACAGTGGGGAT**CG**CATAACCAGTGACAAAGCTGA**CG** TTCTGCTGAATATAGGCAATG (SEQ ID NO: 9)

SEQ ID NO: 10

Part of the nucleotide sequence (DNA, F strand) of the *SLC15A4* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

GAAAA**CG**CTCTGGCCACAGAGGAAGACCACAAAAGCAAGGC**CG**A**CG**CAGAC AGTGGGGAT[**CG**]CATAACCAGTGACAAAGCTGA**CG**TTCTGCTGAATATAGGC AATGCCACCTAA**CG**ACAGGA (SEQ ID NO: 10)

SEQ ID NO: 11

Part of the nucleotide sequence (DNA, F strand) of the *BCL11B* gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

CACAGCCTGACACCAC**CG**AAGTTTC**CG**GGTGCC**CG**GATGCACAGC**CG**AGCT GCCTGGTGC[**CG**]CTAGAACATCAGAGGACTCTAAGATGCAC**CG**AA**CG**CCACC ATGGG**CG**GTTATGGAAGAGA (SEQ ID NO: 11)

SEQ ID NO: 12

Part of the nucleotide sequence (DNA, R strand) of the CD4 gene. CpG-dinucleotide sites in close proximity to the CpG site presented by the Illumina Infinium HumanMethylation450 BeadChip (Illumina, San Diego, USA), which is printed in bold letters and put in squared brackets, are underlined and printed in bold letters:

ACAGCCTGGTCAGACATGG**CG**CTACCACTAATGGAATCTTTCTTGCCATCTTT TTCTTGC[**CG**]CTTAACAGTGGCAGTGACAGTTTGACTCCTGATTTAAGCCTGA TTCTGCTTAACTTTTTC (SEQ ID NO: 12)

SEQ ID NO: 13

Part of the nucleotide sequence (DNA, F strand) of the *CD8A* gene. CpG-dinucleotide site in close proximity to the CpG site presented by the Illumina Infinium HumanMethylation450 BeadChip (Illumina, San Diego, USA), which is printed in bold letters and put in squared brackets, is underlined and printed in bold letters:

GATGATGGCCAGATTTGGGGGTGTACTGCTTTAAAATAGACTAGTTCCATCTT AGTCTCA[**CG**]GAAATCAGCTTGGGGGCCTTCTAGCCCTGCAGCTCAGAAAAG TGTCAGCCAGTGGGGTGG (SEQ ID NO: 13)

The invention further concerns the use of an artificial nucleic acid molecule for determining at least a part of the cellular composition of blood according to the method according to the invention as described above, in which the nucleic acid molecule comprises at least one nucleotide sequence selected from the group consisting of:

a) a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13;
b) a nucleotide sequence which differs from the nucleotide sequence of a) by replacement of at most 10 % of the nucleotides, preferably at most 5 % of the nucleotides, but for the CpG-dinucleotide(s); and
c) a nucleotide sequence which corresponds to the complementary strand of the nucleotide sequence of a) or b).

According to the invention, the methylation level of the specific region may be determined, for example, by methylation specific PCR, sequence analysis of bisulfite-treated DNA, CHIP-sequencing (Illumina Human Methylation BeadChip Technology), Methyl-CAP-sequencing, Next-Generation-Sequencing, DNA methylation analysis with digital PCR, COBRA-Assay and methylation-specific restriction patterns. Alternatively, it is possible to determine the methylation level by MassARRAY assay. It is preferred that the methylation status is determined by pyrosequencing of bisulfite-treated DNA as described in more detail herein below, and which allows identifying whether a specific CpG-dinucleotide was methylated or not, and thereby provides an accurate value for the percentage of methylated CpG-dinucleotides of a given CpG-dinucleotide.
The invention is further described in detail with reference to the exemplary figures and tables.

Brief description of the figures

**Figure 1** shows different representations concerning the selection of cell type-specific CpG sites for the method according to the invention ("Epi-Blood-Count"). (A) Schematic presentation of the work-flow that led to identification of CpG sites that can discern hematopoietic subsets. (B) Scatterplot to exemplarily depict selection criteria for the CpG site for granulocytes (cg05398700): i) high difference between mean β-values in granulocytes (gran.) and the mean β-values of all other cell types in DNAm profiles of purified cell types (GSE35069) (Reinius et al., 2012); and ii) a low variance (var.) of β-values within the granulocytes and within the other hematopoietic subsets (rest). (C) Differences in β-values across blood cell types are exemplarily demonstrated for cg05398700 in reference dataset of Reinius et al. (GSE35069) (Reinius et al., 2012), and (D) in the validation set of Zilbauer et al. (E-MTAB-2145) (Zilbauer et al., 2013). (E) In analogy, the other cell-type specific CpGs of the Epi-Blood-Count were selected. DNAm levels are depicted for each cell type-specific CpG in comparison to all other hematopoietic subsets (GSE35069).

**Figure 2** shows diagrams representing the "Epi-Blood-Count" of granulocytes, lymphocytes, and monocytes. Peripheral blood samples of a training set (A; n = 60) and of an independent validation set (B; n = 44) were analyzed with a Sysmex XN-9000 hematology analyzer and DNAm was analyzed by pyrosequencing at the three CpGs for granulocytes, lymphocytes, and monocytes. Based on the DNAm levels, the Epi-Blood-Count was calculated (NNLS) and the results revealed high correlation with flow cytometric measurements. Performance of the Epi-Blood-Count was compared with other methods for blood counts in an additional validation set of blood samples that was analyzed with (C) a Coulter Counter (ACT II Diff Counter from Beckmann Coulter; n = 24) and/or (D) by microscopic analysis of blood smears and manual counting by a trained operator (n = 66). (E) The correlation of results with conventional established methods was compared for those samples that were analyzed with Coulter Counter and by manual counting (n = 20). (F) To determine how the results of the Epi-Blood-Count are affected by long-term storage (without freezing), an additional set of blood samples (n = 10) that was initially analyzed by manual counting (at day 0) and by DNAm analysis at the three relevant CpGs after storage for 7 days at 4°C was used. Pearson correlation coefficient (R) and mean absolute deviation (MAD) between the measurements were calculated for each cell type and for all cells (black).

**Figure 3** shows diagrams representing leukocyte differential counts with five and six CpG sites. Leukocyte differential counts were determined in the validation set I by combination of Sysmex XN-9000 hematology analyzer and immunophenotypic analysis with FACSCalibur (n = 44). The conventional LDCs were compared with Epi-Blood-Count results that were either determined based on DNAm analysis by pyrosequencing of (A) five CpGs (including a CpG site for all T-cells), or (B) six CpGs to further discriminate between CD4+ and CD8+ T-cells. The models for the Epi-Blood-Count (NNLS) were previously generated on an independent training set. R = Pearson correlation coefficient; MAD = mean absolute deviation.

**Figure 4** shows (A) a simplified hierarchical structure of hematopoietic differentiation. Deconvolution algorithms - including Epi-Blood-Count - are based on estimates for individual subsets that need to sum up to 100% of the cells. According to this stipulation, we used three versions of the Epi-Blood-Count that are either based on three CpGs (granulocytes, lymphocytes, and monocytes); five CpGs (granulocytes, T-cells, B-cells, NK-cells, and monocytes); or six CpGs (granulocytes, CD4+ T-cells, CD8+ T-cells, B-cells, NK-cells, and monocytes). Figure 4 further shows (B-H) scatterplots concerning the selection of relevant CpG sites for each hematopoietic subset. The plots depict DNAm analysis of the dataset of Reinius and coworkers (GSE35069). In analogy to granulocytes (Fig. 1B), we selected CpGs for each hematopoietic subset (mon = monocytes; lym = lymphocytes; B-cells; NK-cells; T-cells; CD4 = CD4+ T-cells; and CD8 = CD8+ T-cells). Selected CpGs and the corresponding gene are indicated (arrows).

**Figure 5** shows diagrams representing DNAm levels of selected CpG sites in different cell types of blood. The DNAm levels (β-values) are depicted for all cell type-specific CpGs in the reference datasets of Reinius and coworkers (GSE35069). Each bar corresponds to one DNAm profile. The relevant CpGs for each cell type are indicated (corresponding genes in brackets). WB = whole blood; PBMC = peripheral blood mononuclear cells.

**Figure 6** illustrates the correlation of blood counts and DNAm levels at individual CpGs. Leukocyte differential counts in peripheral blood samples of the training set (n = 60) were analyzed with a Sysmex XN-9000 (A-C) and for some cell types by additional immunophenotypic analysis with a FACSCalibur (D-H). DNAm at the cell type-specific CpG sites (as indicated) were analyzed by pyrosequencing. Notably, even DNAm levels at the individual CpGs revealed overall a relatively high correlation with the predetermined cell numbers (R = Pearson correlation coefficient). The linear regression formulas are indicated for each cell type - these formulas were subsequently used to calculate the fractions of hematopoietic subsets in validation set I (see **Fig. 8 and Fig. 13**).

**Figure 7** shows diagrams illustrating that DNAm levels of relevant CpGs are not affected by age and gender. The pyrosequencing results of the cell type-specific CpGs in blood samples of the HELPcB program (training set and validation set I) were correlated to either donor age (A; 110 samples) or gender (B; 97 samples). Only two CpGs (associated with CD4+ T-cells (cg05044173) and monocytes (cg10480329) revealed a moderate but significant association with age, and this correlates with generally observed age-associated changes (n.s. = not significant). In addition, this was validated on genome-wide DNAm profiles from peripheral blood (450k data of Hannum and coworkers (GSE40279); not depicted). R = Pearson correlation coefficient.

**Figure 8** shows estimates for blood counts based on linear regression formulas of individual CpGs. Blood samples of the validation set I (n = 44) were analyzed by pyrosequencing at the three CpGs related to granulocytes, lymphocytes, and monocytes. The DNAm levels were implemented into the linear regression formulas of figure S3 to estimate the percentages of individual hematopoietic subsets. These predictions were highly correlated with blood cell counts performed with the Sysmex XN-9000 hematology analyzer. MAD = mean absolute deviation; R = Pearson correlation coefficient.

**Figure 9** illustrates the deconvolution of DNAm levels based on cell counts in peripheral blood. The DNAm levels at the three CpGs for granulocytes (WDR20), lymphocytes (FYN), and monocytes (CENPA) were analyzed by pyrosequencing in blood samples of the training set (n = 60). If no measurements of DNAm levels in purified hematopoietic subsets are available, it is possible to use a reverse approach of the non-negative least-square (NNLS) linear model. The heat map compares the β-values of the reference dataset of Reinius and coworkers derived from purified subsets (GSE35069) and the estimated DNAm levels based on the reverse application of the NNLS approach with the 60 pyrosequencing measurements. Overall, the β-values and estimated DNAm levels of purified subsets are quite similar.

**Figure 10** shows a MassARRAY analysis of the "Epi-Blood-Count". The DNAm levels at the three relevant CpGs for granulocytes, lymphocytes, and monocytes were alternatively analyzed in the 41 fresh blood samples of validation set III with MassARRAY technology. These measurements are based on a sensitive MALDI-TOF mass spectrometer, allowing larger reads and analysis in 96-well or 384-well format. Overall, the results correlated with the previously determined epigenetic cell counts based on pyrosequencing measurements (PSQ), but there is a clear offset in the different methods that needs to be taken into consideration.

**Figure 11** shows a diagram representing an "Epi-Blood-Count" on frozen blood samples. To determine if DNAm levels are affected by long-term cryopreservation of blood samples, we analyzed the Epi-Blood-Count in 41 fresh blood samples of validation set III. 150 μl aliquots of these samples were frozen and stored for three months before reanalyzed of the relevant DNAm levels. There was no systematic offset and reproducibility was very high in cryopreserved samples.

**Figure 12** shows a diagram representing an analysis of cell-free DNA in human serum by the "Epi-Blood-Count".

So far it has not been possible to determine the source of free circulating DNA in serum samples. Cell counts (granulocytes, lymphocytes, and monocytes) were determined in whole blood (WB) by manual counting. In parallel, serum was isolated and analyzed by pyrosequencing for the three relevant CpGs. Epi-Blood-Count results may indicate that free circulating DNA in serum is primarily derived from granulocytes, because this fraction is generally overestimated. This may be anticipated because granulocytes have a much shorter half-life than lymphocytes in peripheral blood. However, it needs to be taken into account that circulating DNA might also stem from non-hematopoietic cells and this cannot be addressed by the Epi-Blood-Count. MAD = mean absolute deviation; R = Pearson correlation coefficient.

**Figure 13** shows estimates for leukocyte differential counts with linear regression of individual CpGs. Leukocyte differential counts (as determined by Sysmex XN-9000 and FACSCalibur) in 44 blood samples of the validation set I were estimated based on the individual linear regression formulas for five CpGs (A) or six CpGs (B). Pyrosequencing results were implemented into the linear regression formulas of figure S3 (training set) to estimate the percentages of individual hematopoietic subsets. Predictions for all hematopoietic subsets correlated with conventional blood counts. MAD = mean absolute deviation; R = Pearson correlation coefficient.

**Figure 14** shows a diagram representing an "Epi-Blood-Count" for five or six hematopoietic subsets. The heat maps compare the β-values of the relevant CpGs in the reference DNAm datasets from Reinius et al. (GSE35069) and estimated DNAm levels based on the reverse approach of NNLS using the pyrosequencing results of the training set (n = 60; in analogy to figure S6). This analysis was performed for the signatures with either five (A) or six (B) CpG sites. These estimates of DNAm values were subsequently implemented into the NNLS matrix to estimate the proportions of cell types in the same training dataset (C+D), or in an independent validation set II (E+F). Please note that the systematic underestimation of granulocytes and the overestimation of other subsets in the validation set II are probably due to different methods for conventional blood counts (Sysmex XN-9000 versus Coulter Counter). MAD = mean absolute deviation; R = Pearson correlation coefficient.

**Figure 15** shows a diagram representing an "Epi-Blood-Count" analysis after storage of blood at 4°C for 7 days. Epi-Blood-Count based on (A) five and (B) six CpG sites to estimate proportions of granulocytes, T-cells, CD4+ T-cells, CD8+ T-cells, B-cells, NK-cells, and monocytes in samples stored for seven days at 4°C. MAD = mean absolute deviation; R = Pearson correlation coefficient.

Description of exemplary and advantageous embodiments of the invention

**Identification of individual CpG sites to discern hematopoietic subsets**

For selection of candidate CpGs DNAm data of purified granulocytes, CD4+ T-cells, CD8+ T-cells, B-cells, NK-cells, and monocytes (GSE35069) that has been published by Reinius and coworkers (Reinius et al., 2012) were utilized. For each of these cell types CpG sites were selected that facilitate best discrimination based on the following two criteria: i) highest difference in mean β-value of the subset and the mean β-value of all other hematopoietic cell types; and ii) low variance of β -values across different samples of the corresponding subset **(Fig. 1A).** This analysis was initially performed for granulocytes **(Fig. 1B and 1C)** and then repeated for the other cell types **(Fig. 4 and 5). Figure 1C** shows that the tested CpG-dinucleotide of the *WDR20* gene has a lower methylation (DNAm) level in granulocytes as in the other cell types. That is, cg05398700 is hypomethylated in granulocytes but has high(er) methylation levels in lymphocytes (T-cells, B-cells, and NK-cells), monocytes, whole blood, and PBMCs (peripheral blood mononuclear cells). **Figure 4** illustrates the selection of cell type-specific CpGs for various hematopoietic subsets. **Figure 5** shows that all tested CpG-dinucleotides that are indicative of a specific cell type have lower methylation (DNAm) levels in this specific cell type as in the other cell types. That is, cg17587997 is hypomethylated in lymphocytes (T-cells, B-cells, and NK-cells) but has high(er) methylation levels in granulocytes, monocytes, whole blood, and PBMCs (peripheral blood mononuclear cells), cg16452866 is hypomethylated in T-cells (CD4+ T-cells and CD8+ T-cells) but has high(er) methylation levels in granulocytes, B-cells, NK-cells, monocytes, whole blood, and PBMCs, cg05044173 is hypomethylated in CD4+ T-cells but has high(er) methylation levels in CD8+ T-cells, granulocytes, B-cells, NK-cells, monocytes, whole blood, and PBMCs, cg25939861 is hypomethylated in CD8+ T-cells but has high(er) methylation levels in CD4+ T-cells, granulocytes, B-cells, NK-cells, monocytes, whole blood, and PBMCs, cg02665297 is hypomethylated in B-cells but has high(er) methylation levels in T-cells (CD4+ T-cells and CD8+ T-cells), granulocytes, NK-cells, monocytes, whole blood, and PBMCs, cg13617280 is hypomethylated in NK-cells but has high(er) methylation levels in T-cells (CD4+ T-cells and CD8+ T-cells), granulocytes, B-cells, monocytes, whole blood, and PBMCs, and cg10480329 is hypomethylated in monocytes but has high(er) methylation levels in T-cells (CD4+ T-cells and CD8+ T-cells), granulocytes, B-cells, NK-cells, whole blood, and PBMCs. Furthermore, we combined DNAm profiles of B-cells, T-cells, and NK-cells to identify CpGs that reflect the entire lymphocyte population; and of CD4+ and CD8+ T-cells as a surrogate for the entire T-cell population. Best performing CpG sites were validated on a second dataset of purified leukocyte subsets (E-MTAB-2145; **Fig. 1D**) (Zilbauer et al., 2013).

For granulocytes we selected a CpG site in the gene WD repeat domain 20 (WDR20; cg05398700). Notably, CpGs with highest discriminatory power for CD4+ T-cells and CD8+ T-cells are linked to the genes CD4 (cg05044173) and CD8A (cg25939861), respectively. Furthermore, the selected CpG site for lymphocytes was in the FYN Proto-Oncogene (FYN; cg17587997); for T-cells in B-cell lymphoma/leukemia 11B (BCL11B; cg16452866) associated with B-cell malignancies; for B-cells in WD repeat domain, phosphoinositide interacting 2 (WIPI2; cg02665297) that is involved in maturation of phagosomes; for NK-cells in solute carrier family 15 member 4 (SLC15A4; cg13617280) that has been implicated in systemic lupus erythematosus; and for monocytes in centromere protein A (CENPA; cg10480329; **Fig. 1E**). Thus, our straight-forward procedure for selection of biomarkers identified CpGs that are associated with genes of important function in the corresponding cell types.

Subsequently, we analyzed if DNAm at our candidate CpGs correlates with the fractions of hematopoietic subsets in blood. To this end, we established pyrosequencing assays for the respective CpG sites and analyzed 60 peripheral blood samples. The percentage of leukocytes - as determined with a Sysmex XN-9000 hematology analyzer - correlated very well with DNAm at the respective CpG sites for granulocytes (R = -0.91), lymphocytes (R = -0.91), and monocytes (R = -0.74). Furthermore, immunophenotypic measurements correlated for T-cells (R = -0.73), CD4+ T-cells (R = -0.41), CD8+ T-cells (R = -0.88), B-cells (R = - 0.66), and NK-cells (R = -0.30; **Fig. 6**). Thus, DNAm at our unique candidate CpGs reflects the frequency of corresponding cell types in blood. Furthermore, we excluded that our candidate CpGs were associated with aging or gender **(Fig. 7).** There was only a moderate age-associated decline in DNAm at the CpGs for CD4+ T-cells and monocytes and this may correspond to the commonly observed decline of these cell populations upon aging.

**Deconvolution of granulocytes, monocytes, and lymphocytes with three CpGs**

The fractions of granulocytes, monocytes, and lymphocytes are routinely determined with automated analyzers or manual microscopic counting. To evaluate if such results can be recapitulated by the three corresponding CpGs we measured DNAm levels in 44 independent blood samples by pyrosequencing. Initially, the percentages of cells were simply calculated based on the linear regression formulas of the subsets in the training set **(Fig. 6).** In comparison to measurements of the Sysmex XN-9000 analyzer the linear regression models based on three CpGs revealed extremely high correlation (R = 0.99 across all cell types). The mean absolute deviation (MAD) was only 3.2% for granulocytes, 2.2% for lymphocytes, and 1.4% for monocytes **(Fig. 8).**

Alternatively, we integrated the DNAm levels of the three CpGs into a non-negative least-squares (NNLS) linear regression model. This model was trained on 60 blood samples of the training set and is subsequently termed "Epi-Blood-Count". The NNLS linear regression approach does not depend on an a-priori database of cell type-specific DNAm reference profiles. Either way, the estimated DNAm levels based on deconvolution of 60 blood samples of the training set were very similar to the β-values of DNAm profiles of purified subsets (Reinius et al., 2012) **(Fig. 9).** This approach gave similar accuracies as the linear models based on individual CpGs for granulocytes, lymphocytes, and monocytes **(Fig. 2A and 2B).**

Our Epi-Blood-Count was trained on cell counts that were determined with the Sysmex XN-9000 analyzer - however, there are notoriously differences between cell counting systems. Therefore, we applied the Epi-Blood-Count on a second validation set (in total 70 blood samples) that were either measured with a Coulter Counter (ACT II Diff Counter, Beckman Coulter; n = 24), and/or by manual counting of blood smears by trained laboratory staff (n = 66). Coulter Counter results revealed very high correlation with the Epi-Blood-Count, albeit granulocytes were in average 4.4% underestimated and lymphocytes were 5.5% overestimated, indicating that there might be a systemic deviation between the two analyzers **(Fig. 2C).** The correlation of manual blood counts and Epi-Blood-Count was slightly lower **(Fig. 2D).** However, direct comparison of Coulter Counter results and manual counting revealed lower correlations, too **(Fig. 2E).** On the other hand, it is also possible to determine site-specific DNAm patterns with other techniques: predictions of pyrosequencing and MassARRAY analysis were correlated, but there was a systematic offset that needs to be taken into consideration **(Fig. 10).**

Long-term storage of blood affects LDCs and therefore, we tested if the Epi-Blood-Count is also applicable after storage of blood samples for seven days at 4°C. Overall, the results correlated with manual counting at day 0, but the numbers of granulocytes were in average 9.1% underestimated, whereas lymphocytes were 7.2% overestimated **(Fig. 2F).** The same trend is observed with automated analyzers upon storage of blood samples for more than 72 hours. Either way, Epi-Blood-Count results at day 7 revealed a very high correlation with manual counting at dO.

On the other hand, it is possible to freeze fresh blood samples and store them for months without affecting the results by the Epi-Blood-Count. To test this, we used a third validation set (n = 41) that was either analyzed by Epi-Blood-Count in aliquots of fresh blood and after freeing the blood samples for three months. DNAm levels at the relevant CpGs were not affected by long-term cryopreservation **(Fig. 11).** Another advantage of the Epi-Blood-Count is that it is applicable to very low amounts of DNA. We have exemplarily used this approach to measure cell-free circulating DNA (cfDNA) in serum. The results indicated that cfDNA in serum is particularly derived from granulocytes, which indeed have a very short half-life **(Fig. 12).**

**Additional classification of lymphocyte subsets**

Subsequently, we analyzed whether the Epi-Blood-Count can be further extended to classify subsets of lymphocytes. To this end, the DNAm levels at the candidate CpGs for B-cells, NK-cells, CD4+ T-cells, CD8+ T-cells, and T-cells (CD4+ and CD8+ combined) were analyzed by pyrosequencing in the 60 blood samples of the training set. Since the percentages of these subsets correlated well with DNAm levels **(Fig. 6)** it was again possible to determine the LDCs based on linear regression formulas. However, some hematopoietic subsets were estimated to have negative values **(Fig. 13).** Alternatively, the corresponding immunophenotypic measurements of the training set were imputed to generate NNLS regression models for either five CpGs (all T-cells) or six CpGs (separate analysis of CD4+ and CD8+ T-cells). With this deconvolution approach the estimated DNAm levels for each hematopoietic subset closely resembled the β-values of the purified subsets in DNAm profiles (Reinius et al., 2012) **(Fig. 14A and 14B).** The 5 CpG and 6 CpG Epi-Blood-Count models were then tested on the training set **(Fig. 14C and 14D)** and on two independent validation sets **(Fig. 3** and **Fig. 14E and 14F).** Immunophenotypic analysis and Epi-Blood-Count revealed a clear correlation: across all cell types the correlation coefficients for the 5 CpG and 6 CpG models were R = 0.99 and R = 0.98 with average MADs of 2.8% and 3.1%, respectively. Furthermore, the Epi-Blood-Count is applicable to blood samples that have been stored at 4°C for seven days **(Fig. 15).**

Analysis of DNAm patterns in peripheral blood holds enormous clinical potential, which has so far hardly been utilized. We demonstrate that site-specific analysis at individual CpG sites facilitates relative quantification of leukocyte subpopulations. Analytic performance is traditionally evaluated by precision (or random error), accuracy (or systematic error), and clinical sensitivity. It was somewhat unexpected that analysis of DNAm at few individual CpG sites reached similar precision and accuracy as the well-established conventional methods (Buttarello and Plebani, 2008).

The previously published algorithms for LDCs are based on DNAm profiles that were generated with Illumina BeadChip microarrays (Accomando et al., 2014; Houseman et al., 2012; Teschendorff et al., 2017). Such larger signatures have the big advantage to combine a multitude of CpGs, which generally increases the precision of epigenetic signatures (Koestler et al., 2013). On the other hand, the precision of DNAm levels at individual CpGs is higher in pyrosequencing data as compared to β-values on Illumina BeadChips (BLUEPRINT consortium, 2016; Lin et al., 2016). Furthermore, analysis of genome-wide DNAm profiles takes longer and it is relatively expensive. This is also the reason, why the number of available DNAm profiles with matched flow cytometric analysis is still relatively low. Reinius et al. provided flow cytometric analysis for six DNAm profiles (Reinius et al., 2012) and Absher and colleagues provided 44 DNAm profiles with conventional LDCs (Absher et al., 2013). Notably, the precision of genome-wide algorithms on these datasets was similar to the performance of the Epi-Blood-Count in our cohorts **(Table 1 and 2)** (Waite et al., 2016). Furthermore, Koestler and coworkers compared their predictions of cell types with complete blood counts that were analyzed on an Advia 70 hematology system and the correlation for monocytes (R = 0.60) and lymphocytes (R = 0.61) was not better than our 3 CpG Epi-Blood-Count (Koestler et al., 2013). A recent study indicated that non-constrained methods, such as support vector regressions or robust partial regression, might further improve the accuracy of deconvolution methods for DNAm profiles (Teschendorff et al., 2017). Either way, site-specific analysis of individual cell type-specific CpGs is better applicable to daily routine in clinical diagnostics.

Relative quantification of cell types is of relevance for clinical diagnostics. Analysis of DNAm levels at individual CpGs can reflect relative abundance of hematopoietic subsets. Immunophenotypic analysis is based on individual cell type-specific epitopes, too. Notably, several candidate CpGs of the Epi-Blood-Count are related to the same genes addressed in immunophenotypic analysis. Our Epi-Blood-Count has various advantages over the well-established conventional methods: i) blood can be frozen after sampling for long-term storage, shipment, and subsequent analysis; ii) it is applicable to relatively small volumes of blood; and iii) DNAm levels at individual CpGs provide an absolute measure that may facilitate better standardization between labs than immunophenotypic analysis by flow cytometry. It is however unlikely that epigenetic analysis of LDCs will completely replace the conventional flow cytometric methods, because it cannot address erythrocytes and thrombocytes, which hardly contain DNA.

Methods

**Selection of candidate CpGs**

For selection of cell type-specific CpG sites we used DNAm profiles of purified leukocyte subsets that were generated on the Illumina Infinium HumanMethylation450 BeadChip platform (GSE35069) (Reinius et al., 2012). We utilized β-values, ranging from 0 to 1, which provide a measure for each CpG site represented on the array that roughly corresponds to the percentage of DNAm. CpG sites on X and Y chromosomes were excluded. Candidate CpGs were selected based on i) highest difference between the mean β-value of one purified leukocyte subset and the mean β-value of all other subsets; and ii) low variation of β -values within purified subsets. Best performing CpG sites were further evaluated on an independent dataset from Zilbauer et al. (E-MTAB-2145) (Zilbauer et al., 2013), which also provides DNAm data of purified leukocyte subsets.

For selection of CpGs for the cellular quantification we identified genomic regions that were generally methylated in all blood subsets. To this end, we used the following DNAm datasets that were all analyzed on the HumanMethylation450 BeadChip (accession numbers for NCBI gene expression omnibus (GEO) are provided): i) purified leukocyte subsets (GSE35069) (Reinius et al., 2012), (E-MTAB-2145) (Zilbauer et al., 2013); whole blood from healthy donors (GSE32148), (GSE41169); and DNAm profiles of blood disorders (acute myeloid leukemia: TCGA, and (GSE58477, and GSE62298); myelodysplastic syndrome: (GSE51758); myeloproliferative neoplasms: own data; B-cell lymphoma: (GSE37362); acute lymphoblastic leukemia: (GSE69954)). Mean β-values were calculated for each datasets and all CpGs. Subsequently, we selected one candidate CpG site (LSM14B; cg06096175) that was consistently highly methylated in each dataset (β-value > 0.975).

### Blood samples

Peripheral blood samples for the training set (60 samples) and for the validation set I (44 samples) were obtained from the HELPcB program (Health Effects in High-Level Exposure to PCB) (Schettgen et al., 2012). The study was approved by the local ethics committee of the Medical Faculty of the RWTH Aachen University (EK 176/11). Peripheral blood samples for validation set II (70 samples) and validation set III (41 samples), as well as serum samples (18 samples) were obtained from the Department of Hematology and Oncology and from the Department of Transfusion Medicine according to the guidelines specifically approved by the local ethics committee of RWTH Aachen University (EK 099/14).

### Conventional analysis of blood counts

Blood samples from the HELPcB program were analyzed with the Sysmex XN-9000 hematology analyzer (Sysmex Deutschland GmbH, Norderstedt, Germany). Lymphocyte phenotyping was performed as described before (Haase et al., 2016). In brief, EDTA anti-coagulated whole blood was incubated for 20 minutes at room temperature with fluorescently labeled antibody pairs (CD3/CD4, CD3/CD8, CD3/CD19, CD3/CD16+CD56) and isotype matched controls (IgG1 FITC/IgG2a PE, all from Becton Dickinson, Heidelberg, Germany). Erythrocytes were lysed with BD FACS lysing solution according to the manufacturer's instructions and leukocytes were analyzed by flow cytometry on a FACSCalibur using the BD Simulset software for data acquisition and analysis (Becton Dickinson). LDCs of validation set II were determined either i) with an automated hematology analyzer (Coulter AcT diff2, Beckman Coulter, Brea, California, USA); ii) by microscopic analysis of blood smears; and/or iii) by immunophenotyping and flow cytometric analysis on a Navios flow cytometer (Beckman Coulter) to get proportions of T-cells, CD4+ T-cells, CD8+ T-cells, B-cells, and NK-cells, as indicated in the text.

### Isolation of DNA and bisulfite conversion

Genomic DNA was isolated from blood with the QIAamp DNA Mini Kit (Qiagen, Hilden, Germany). Genomic DNA of serum was isolated from serum tubes (S-Monovette; Sarstedt, Nümbrecht, Germany) after one-hour incubation at room temperature and centrifugation for ten minutes at 2,000 x g. DNA was subsequently isolated from 1 ml serum with the PME free-circulating DNA Extraction Kit (GS/VL system; Analytik Jena, Jena, Germany) with addition of carrier RNA according to the manufacturer's instructions. Either 1 μg of DNA from peripheral blood or the complete DNA sample from serum was bisulfite-converted with the EZ DNA Methylation Kit (Zymo Research, Irvine, CA, USA).

### Pyrosequencing

Specific regions covering the CpG sites cg05398700 (WDR20), cg17587997 (FYN), cg16452866 (BCL11B), cg05044173 (CD4), cg25939861 (CD8A), cg02665297 (WIPI2), cg13617280 (SLC15A4), and cg10480329 (CENPA) were amplified by PCR (Eppendorf Mastercycler 5341; Eppendorf AG). Primers were designed with the Pyrosequencing Assay Design Software 1.0 (Biotag AB, Uppsala, Sweden) and they are provided in **Table 3.** Pyrosequencing was then performed on a PyroMark Q96 ID System using region specific sequencing primers and results were analyzed with the PyroMark Q CpG software (Qiagen).

## MassARRAY analysis

Alternatively, we used MassARRAY for site-specific analysis of DNAm levels. Amplicons were designed with the Sequenom's EpiDESIGNER software **(Table 4).** Converted DNA was amplified by PCR using the HotStart Plus PCR Master Mix (Qiagen). Unincorporated dNTPs were neutralized using shrimp alkaline phosphatase (Agena Bioscience, San Diego, CA, USA). Subsequently, 10 μl of PCR product was in vitro transcribed and cleaved in a base-specific (U-specific) manner using RNase A (T-Cleavage MassCleave Kit; Agena Bioscience). The cleaved products were then analyzed by the MALDI-TOF mass spectrometer (MassARRAY Analyzer 4 System; Agena Bioscience, Hamburg, Germany). Measurements were performed at Varionostic GmbH (www.varionostic.de; Ulm, Germany).

## Combination of DNAm levels for Epi-Blood-Count

Linear regression of DNAm levels at individual CpG sites was initially determined in 60 samples of the HELPcB training set. The regression formulas (indicated in fig. S3) were then used to estimate cell counts in a validation set. Alternatively, we used a deconvolution approach that combines DNAm data of various cell types (three, five, or six CpGs). The Epi-Blood-Count can be represented by a matrix W of size f x k (f: number of CpGs (features); k: number of cell types). The methylation data of the blood samples are represented by a matrix V of size f x n (n: number of blood samples) and are modeled as a linear combination of the purified cell types W, with their mixture proportions H (k x n matrix - each of the n columns corresponds to the mixture proportion of the respective blood sample (same column in V)): $V \approx WH$

For estimation of H, a non-negative least-squares (NNLS) approach is used to avoid negative mixture proportions. For implementation purposes, we use the multiplicative update rule of Lee et al. (Lee and Seung, 2001) to determine H:

$$H_{a\mu}^{j+1} = H_{a\mu}^{j} \frac{(W^T V)_{a\mu}}{(W^T W H^j)_{a\mu}}$$

Here, j is the iteration index, WT indicates the transpose of matrix W, and $\alpha$ and $\mu$ are the row and column indices, respectively. For a unique estimation of H, it is required to have at least as many CpG sites as cell types, i.e. $f \geq k$ (assuming that the rows of W are linearly independent). Once leukocyte proportions were calculated, we added the proportions together and adjusted them to a total sum of 100%.

If no measurements from purified cell types are available, it is possible to use the reverse approach, estimating matrix W from H and V, given a set of peripheral blood samples with available cell counts (H) and methylation data (V). In order to do so, we use the respective iterative formula (Lee and Seung, 2001) for estimating W:

$$W_{ia}^{j+1} = W_{ia}^{j} \frac{(VH^T)_{ia}}{(W^j H H^T)_{ia}}$$

## Statistical Analysis

Mean average deviation (MAD), mean standard error (MSE), Pearson correlation coefficient (R), linear regression, and Student's t test were calculated in MS Excel. P values < 0.05 were considered as statistically significant.

**Table 1:** Correlations of Epi-Blood-Count *versus* deconvolution algorithms on DNAm profiles.

| | Epi-Blood-Count | Two-Stage Model [Waite et al.] | | EstimateCellCounts [Jaffe and Irizarry] | |
|---|---|---|---|---|---|
| **Cell Type** | Validation set I [6 CpGs] | Absher WB | Reinius WB | Absher WB | Reinius WB |
| Granulocytes | 0.85 | 0.75 | 0.70 | 0.75 | 0.59 |
| CD4+ T-Cells | 0.64 | 0.74 | 0.71 | 0.75 | 0.78 |
| CD8+ T-Cells | 0.65 | 0.61 | 0.89 | 0.66 | 0.60 |
| B-Cells | 0.63 | 0.92 | 0.64 | 0.93 | 0.59 |
| NK-Cells | 0.36 | 0.81 | 0.91 | 0.82 | 0.87 |
| Monocytes | 0.48 | 0.58 | 0.82 | 0.59 | 0.64 |
| Average | 0.60 | 0.74 | 0.78 | 0.75 | 0.68 |

This table compares Pearson correlation coefficients (R) of predicted cell fractions and conventionally determined LDCs. The results of the Epi-Blood-Count are based on 44 blood samples of the validation set I (corresponding to Figure 3B). The Two-Stage Model and EstimateCellCounts were tested on 450k Illumina Bead Chip data of whole blood (WB) samples from the Reinius (GSE35069; n = 6) and the Absher datasets (n = 44). The Pearson correlation coefficients for these algorithms were taken from the publication of Waite et al. (Frontiers in genetics 7, 23; 2016).

**Table 2:** Mean standard error of Epi-Blood-Count *versus* deconvolution algorithms.

| | Epi-Blood-Count | Two-Stage Model [Waite et al.] | | EstimateCellCounts [Jaffe and Irizarry] | |
|---|---|---|---|---|---|
| **Cell Type** | Validation set I [6 CpGs] | Absher WB | Reinius WB | Absher WB | Reinius WB |
| Granulocytes | 0.0019 | 0.0103 | 0.0228 | 0.0109 | 0.0178 |
| CD4+ T-Cells | 0.0035 | 0.0021 | 0.0005 | 0.0025 | 0.0012 |
| CD8+ T-Cells | 0.0011 | 0.0036 | 0.0086 | 0.0019 | 0.0016 |
| B-Cells | 0.0004 | 0.0004 | 0.0012 | 0.0014 | 0.0025 |
| NK-Cells | 0.0018 | 0.0004 | 0.0001 | 0.0003 | 0.0019 |
| Monocytes | 0.0006 | 0.0007 | 0.001 | 0.0008 | 0.0008 |
| Average | 0.0016 | 0.0029 | 0.0057 | 0.0030 | 0.0043 |

The results of the Epi-Blood-Count are based on 44 blood samples of the validation set I. Mean standard error (MSE) for the Two-Stage Model and EstimateCellCounts algorithms were tested on DNAm profiles of Reinius et al. (GSE35069; n = 6) and the Absher datasets (n = 44). The MSE values were taken from Waite et al. (Frontiers in genetics 7, 23; 2016).

**Table 3:** Primers used in pyrosequencing assays

| Primer | Sequence |
|---|---|
| *WDR20* | |
| Forward | 5'-ACGAAGTTTAAACGAGGGTTATGTTAGGGAGAAG -3' (SEQ ID NO: 14) |
| Reverse | 5'-Biotin-GCCAACAACATTACAAAAATACCTCTTACCA-3' (SEQ ID NO: 15) |
| Sequencing | 5'-AGATTCGGGGAGTTTTAG-3' (SEQ ID NO: 16) |
| *FYN* | |
| Forward | 5'-Biotin-TAGAGGGAGGTTGTTTGGTTATAAATAGTT-3' (SEQ ID NO: 17) |
| Reverse | 5'-CACTTTCCAACATATTAATTATAAAAATAAATACTTTACATA-3' (SEQ ID NO: 18) |
| Sequencing | 5'-CTAATTTACAAACAAATAACAC-3' (SEQ ID NO: 19) |
| *PCYOX1* | |
| Forward | 5'-TTTGTAAAATAGGATTTTTATAGGAATGGAAGTGT-3' (SEQ ID NO: 20) |
| Reverse | 5'-Biotin-TCAAAAAAAAAAAACCAAAATACCAACTAAATTC-3' (SEQ ID NO: 21) |
| Sequencing | 5'-AAGGTATATTTAGAATAAATTA-3' (SEQ ID NO: 22) |
| *RPS6KA2* | |
| Forward | 5'-GTAGTTATGAGTTCGTAATTTTTGATAACGGGAGGAAG-3'(SEQ ID NO: 23) |
| Reverse | 5'-Biotin-CGTCAAAACTACCAAAATACTTTATCGTTCCTACATA-3' (SEQ ID NO: 24) |
| Sequencing | 5'-GTTATAGGGATTGGGATAG-3' (SEQ ID NO: 25) |
| *BCL11B* | |
| Forward | 5'-GGGATTTTGGGTCGACGTAGATGGTAAATAAAG-3' (SEQ ID NO: 26) |
| Reverse | 5'-Biotin-CCCATAATAACGTTCGATACATCTTAAAATCCTCTAAT-3' (SEQ ID NO: 27) |
| Sequencing | 5'-GGGTGTTCGGATGTATAGTCGAGTTG-3' (SEQ ID NO: 28) |
| *CD4* | |
| Forward | 5'-Biotin-GGAATATGTTAAAGTGAAAATGTTAAAGTTAAGGGA-3' (SEQ ID NO: 29) |
| Reverse | 5'-CATATACCCCCAACTACCTCCCACTTC-3' (SEQ ID NO: 30) |
| Sequencing | 5'-AATAAAATCTTTCTTACCATCTT-3' (SEQ ID NO: 31) |
| *CD8A* | |
| Forward | 5'-Biotin-TTTAGTGGTGGAAGTATTGAGGGGGTTAGAG-3' (SEQ ID NO: 32) |
| Reverse | 5'-CACTTTTCTAAACTACAAAACTAAAAAACCCCCAAAC-3' (SEQ ID NO: 33) |
| Sequencing | 5'-CTAAAAAACCCCCAAAC-3' (SEQ ID NO: 34) |
| *WIPI2* | |
| Forward | 5'-TTGGTGGGTAGTTTTTGGGTTTGT-3' (SEQ ID NO: 35) |
| Reverse | 5'-Biotin-CCACCTACCGTTAAATTTTTTCATATCTATAC-3' (SEQ ID NO: 36) |
| Sequencing | 5'-AATTGGTTTTGATAAGTAGATT-3' (SEQ ID NO: 37) |
| *SLC15A4* | |
| Forward | 5'-ATGAAAACGTTTTGGTTATAGAGGAAGATTATAAA-3' (SEQ ID NO: 38) |
| Reverse | 5'-Biotin-TCCTATCGTTAAATAACATTACCTATATTCAACAA-3' (SEQ ID NO: 39) |
| Sequencing | 5'-ATAGAGGAAGATTATAAAAGTAAG-3' (SEQ ID NO: 40) |
| *CENPA* | |
| Forward | 5'-Biotin-ATTATGTTGGTTAGGTTGGTTTTTAATTGTTAATT-3' (SEQ ID NO: 41) |
| Reverse | 5'-AAAATCAAAAAAACACTCCTACCCTTTCTCTTA-3' (SEQ ID NO: 42) |
| Sequencing | 5'-AAACACTCCTACCCTTTCTC-3' (SEQ ID NO: 43) |

**Table 4:** Primers used in MassARRAY assays

| Primer | Sequence (capital letters: gene specific) |
|---|---|
| *WDR20* | |
| Forward | 5'-aggaagagagGTTAGGGTGGGAGTTATTGAGGA-3' (SEQ ID NO: 44) |
| Reverse | 5'-cagtaatacgactcactatagggagaaggctCCAACAACATTACAAAAATACCTCTT-3' (SEQ ID NO: 45) |
| *FYN* | |
| Forward | 5'-aggaagagagGTGATTTGGTTTATTTTTGAGTGAGA-3' (SEQ ID NO: 46) |
| Reverse | 5'-cagtaatacgactcactatagggagaaggctTTCTAAAAACACTAAAAAAACCCCTT-3' (SEQ ID NO: 47) |
| *CENPA* | |
| Forward | 5'-aggaagagagGGGGTTAAGGAAATATTTTTGTTTTT-3' (SEQ ID NO: 48) |
| Reverse | 5'-cagtaatacgactcactatagggagaaggctTCAAACTAATCTCCAACTACCAACC-3' (SEQ ID NO: 49) |

**References**

Absher, D.M., Li, X., Waite, L.L., Gibson, A., Roberts, K., Edberg, J., Chatham, W.W., and Kimberly, R.P. (2013). Genome-wide DNA methylation analysis of systemic lupus erythematosus reveals persistent hypomethylation of interferon genes and compositional changes to CD4+ T-cell populations. PLoS Genet 9, e1003678.

Accomando, W.P., Wiencke, J.K., Houseman, E.A., Nelson, H.H., and Kelsey, K.T. (2014). Quantitative reconstruction of leukocyte subsets using DNA methylation. Genome biology 15, R50.

Adalsteinsson, B.T., Gudnason, H., Aspelund, T., Harris, T.B., Launer, L.J., Eiriksdottir, G., Smith, A.V., and Gudnason, V. (2012). Heterogeneity in white blood cells has potential to confound DNA methylation measurements. PloS one 7, e46705.

BLUEPRINT consortium (2016). Quantitative comparison of DNA methylation assays for biomarker development and clinical applications. Nature biotechnology 34, 726-737.

Buttarello, M., and Plebani, M. (2008). Automated blood cell counts: state of the art. Am J Clin Pathol 130, 104-116.

Haase, H., Fahlenkamp, A., Schettgen, T., Esser, A., Gube, M., Ziegler, P., Kraus, T., and Rink, L. (2016). Immunotoxicity monitoring in a population exposed to polychlorinated biphenyls. International journal of environmental research and public health 13.

Houseman, E.A., Accomando, W.P., Koestler, D.C., Christensen, B.C., Marsit, C.J., Nelson, H.H., Wiencke, J.K., and Kelsey, K.T. (2012). DNA methylation arrays as surrogate measures of cell mixture distribution. BMC bioinformatics 13, 86.

Houseman, E.A., Kim, S., Kelsey, K.T., and Wiencke, J.K. (2015). DNA Methylation in Whole Blood: Uses and Challenges. Current environmental health reports 2, 145-154.

Jaffe, A.E., and Irizarry, R.A. (2014). Accounting for cellular heterogeneity is critical in epigenome-wide association studies. Genome biology 15, R31.

Koestler, D.C., Christensen, B., Karagas, M.R., Marsit, C.J., Langevin, S.M., Kelsey, K.T., Wiencke, J.K., and Houseman, E.A. (2013). Blood-based profiles of DNA methylation predict the underlying distribution of cell types: a validation analysis. Epigenetics 8, 816-826.

Lee, D.D., and Seung, H.S. (2001). Algorithms for non-negative matrix factorization. Adv Neural Inform Process Systems 13, 556-562.

Lin, Q., Weidner, C.I., Costa, I.G., Marioni, R.E., Ferreira, M.R., Deary, I.J., and Wagner, W. (2016). DNA methylation levels at individual age-associated CpG sites can be indicative for life expectancy. Aging (Albany NY) 8, 394-401.

McGregor, K., Bernatsky, S., Colmegna, I., Hudson, M., Pastinen, T., Labbe, A., and Greenwood, C.M. (2016). An evaluation of methods correcting for cell-type heterogeneity in DNA methylation studies. Genome biology 17, 84.

Reinius, L.E., Acevedo, N., Joerink, M., Pershagen, G., Dahlen, S.E., Greco, D., Soderhall, C., Scheynius, A., and Kere, J. (2012). Differential DNA methylation in purified human blood cells: implications for cell lineage and studies on disease susceptibility. PloS one 7, e41361.

Schettgen, T., Gube, M., Esser, A., Alt, A., and Kraus, T. (2012). Plasma polychlorinated biphenyls (PCB) levels of workers in a transformer recycling company, their family members, and employees of surrounding companies. Journal of toxicology and environmental health Part A 75, 414-422.
Teschendorff, A.E., Breeze, C.E., Zheng, S.C., and Beck, S. (2017). A comparison of reference-based algorithms for correcting cell-type heterogeneity in Epigenome-Wide Association Studies. BMC Bioinformatics 18, 105.
Waite, L.L., Weaver, B., Day, K., Li, X., Roberts, K., Gibson, A.W., Edberg, J.C., Kimberly, R.P., Absher, D.M., and Tiwari, H.K. (2016). Estimation of Cell-Type Composition Including T and B Cell Subtypes for Whole Blood Methylation Microarray Data. Frontiers in genetics 7, 23.
Zheng, S.C., Beck, S., Jaffe, A.E., Koestler, D.C., Hansen, K.D., Houseman, A.E., Irizarry, R.A., and Teschendorff, A.E. (2017). Correcting for cell-type heterogeneity in epigenome-wide association studies: revisiting previous analyses. Nat Methods 14, 216-217.
Zilbauer, M., Rayner, T.F., Clark, C., Coffey, A.J., Joyce, C.J., Palta, P., Palotie, A., Lyons, P.A., and Smith, K.G. (2013). Genome-wide methylation analyses of primary human leukocyte subsets identifies functionally important cell-type-specific hypomethylated regions. Blood 122, e52-60.

## Claims

**1.** Method for determining at least a part of the cellular composition of blood, comprising:

- Isolating genomic DNA from at least one blood sample so as to obtain at least one nucleic acid preparation of said blood sample;
- Identifying DNA methylation levels of at least two different specific regions of said nucleic acid preparation, each specific region comprising at least one CpG-dinucleotide, wherein each of said two different specific regions comprises at least one nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13; and
- Determining the proportions of at least two different blood cell types based on the identified methylation levels so as to predict the relative distribution of said at least two different blood cell types in the blood sample, wherein the methylation level of at least one CpG-dinucleotide selected from the group consisting of nucleotides no. 6 and 7, 38 and 39, 49 and 50, 51 and 52, 61 and 62, 80 and 81, 89 and 90, and 112 and 113 of SEQ ID NO: 1 is indicative of the percentage of granulocytes, the methylation level of at least one CpG-dinucleotide selected from the group consisting of nucleotides no. 40 and 41, 45 and 46, 53 and 54, and 61 and 62 of SEQ ID NO: 5 is indicative of the percentage of monocytes, the methylation level of nucleotides no. 61 and 62 of SEQ ID NO: 6 is indicative of the percentage of lymphocytes, the methylation level of at least one CpG-dinucleotide selected from the group consisting of nucleotides no. 15 and 16, 26 and 27, 32 and 33, and 61 and 62 of SEQ ID NO: 2 is indicative of the percentage of neutrophil granulocytes, the methylation level of at least one CpG-dinucleotide selected from the group consisting of nucleotides no. 6 and 7, 21 and 22, 32 and 33, 34 and 35, 54 and 55, 61 and 62, 63 and 64, 81 and 82, 93 and 94, 101 and 102, and 121 and 122 of SEQ ID NO: 3, or selected from the group consisting of nucleotides no. 1 and 2, 12 and 13, 14 and 15, 34 and 35, 41 and 42, 43 and 44, 61 and 62, 73 and 74, 81 and 82, and 101 and 102 of SEQ ID NO: 4 is indicative of the percentage of eosinophil granulocytes, the methylation level of at least one CpG-dinucleotide selected from the group consisting of nucleotides no. 5 and 6, 36 and 37, 42 and 43, 53 and 54, 58 and 59, 61 and 62, and 63 and 64 of SEQ ID NO: 7 is indicative of the percentage of B-cells, the methylation level of at least one CpG-dinucleotide selected from the group consisting of nucleotides no. 25 and 26, 61 and 62, 64 and 65, 81 and 82, and 104 and 105 of SEQ ID NO: 8, or selected from the group consisting of nucleotides no. 22 and 23, 58 and 59, 61 and 62, 77 and 78, and 100 and 101 of SEQ ID NO: 9, or selected from the group consisting of nucleotides no. 6 and 7, 42 and 43, 45 and 46, 61 and 62, 84 and 85, and 115 and 116 of SEQ ID NO: 10 is indicative of the percentage of NK-cells, the methylation level of at least one CpG-dinucleotide selected from the group consisting of nucleotides no. 17 and 18, 26 and 27, 34 and 35, 46 and 47, 61 and 62, 92 and 93, 96 and 97, and 108 and 109 of SEQ ID NO: 11 is indicative of the percentage of T-cells, the methylation level of nucleotides no. 20 and 21, and/or 61 and 62 of SEQ ID NO: 12 is indicative of the percentage of CD4+ T-cells, and the methylation level of nucleotides no. 61 and 62 of SEQ ID NO: 13 is indicative of the percentage of CD8+ T-cells.

**2.** Use of an artificial nucleic acid molecule for determining at least a part of the cellular composition of blood according to the method of claim 1, in which the nucleic acid molecule comprises at least one nucleotide sequence selected from the group consisting of:

a) a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13;
b) a nucleotide sequence which differs from the nucleotide sequence of a) by replacement of at most 10 % of the nucleotides, preferably at most 5 % of the nucleotides, but for the CpG-dinucleotide(s); and
c) a nucleotide sequence which corresponds to the complementary strand of the nucleotide sequence of a) or b).

## Patentansprüche

**1.** Verfahren zur Bestimmung zumindest eines Teils der zellulären Zusammensetzung von Blut, umfassend:

- Isolieren von genomischer DNA aus mindestens einer Blutprobe, um mindestens eine Nukleinsäure-Aufbereitung aus dieser Blutprobe zu erhalten;
- Identifizieren von DNA-Methylierungsgraden von mindestens zwei unterschiedlichen spezifischen Regionen der genannten Nukleinsäure-Aufbereitung, wobei jede spezifische Region mindestens ein CpG-Dinukleotid umfasst, und wobei jede der genannten zwei unterschiedlichen spezifischen Regionen mindestens eine Nukleotidsequenz umfasst, die aus der Gruppe bestend aus SEQ ID NR: 1, SEQ ID NR: 2, SEQ ID NR: 3, SEQ ID NR: 4, SEQ ID NR: 5, SEQ ID NR: 6, SEQ ID NR: 7, SEQ ID NR: 8, SEQ ID NR: 9, SEQ ID NR: 10, SEQ ID NR: 11, SEQ ID NR: 12, and SEQ ID NR: 13 ausgewählt ist; und
- Bestimmen des Verhältnisses zwischen mindestens zwei verschiedenen Blutzelltypen basierend auf den identifizierten Methylierungsgraden, um die relative Verteilung dieser verschiedenen Blutzelltypen in der Blutprobe vorherzusagen, wobei der Methylierungsgrad mindestens eines CpG-Dinukleotids, das aus der Gruppe bestehend aus den Nukleotiden Nr. 6 und 7, 38 und 39, 49 und 50, 51 und 52, 61 und 62, 80 und 81, 89 und 90, und 112 und 113 der SEQ ID NR: 1 ausgewählt ist, den Anteil an Granulozyten anzeigt, der Methylierungsgrad mindestens eines CpG-Dinukleotids, das aus der Gruppe bestehend aus den Nukleotiden Nr. 40 und 41, 45 und 46, 53 und 54, und 61 und 62 der SEQ ID NR: 5 ausgewählt ist, den Anteil an Monozyten anzeigt, der Methylierungsgrad der Nukleotide Nr. 61 und 62 der SEQ ID NR: 6 den Anteil an Lymphozyten anzeigt, der Methylierungsgrad mindestens eines CpG-Dinukleotids, das aus der Gruppe bestehend aus den Nukleotiden Nr. 15 und 16, 26 und 27, 32 und 33, und 61 und 62 der SEQ ID NR: 2 ausgewählt ist, den Anteil an neutrophilen Granulozyten anzeigt, der Methylierungsgrad mindestens eines CpG-Dinukleotids, das aus der Gruppe bestehend aus den Nukleotiden Nr. 6 und 7, 21 und 22, 32 und 33, 34 und 35, 54 und 55, 61 und 62, 63 und 64, 81 und 82, 93 und 94, 101 und 102, und 121 und 122 der SEQ ID NR: 3 oder aus der Gruppe bestehend aus den Nukleotiden Nr. 1 und 2, 12 und 13, 14 und 15, 34 und 35, 41 und 42, 43 und 44, 61 und 62, 73 und 74, 81 und 82, und 101 und 102 der SEQ ID NR: 4 ausgewählt ist, den Anteil an eosinophilen Granulozyten anzeigt, der Methylierungsgrad mindestens eines CpG-Dinukleotids, das aus der Gruppe bestehend aus den Nukleotiden Nr. 5 und 6, 36 und 37, 42 und 43, 53 und 54, 58 und 59, 61 und 62, und 63 und 64 der SEQ ID NR: 7 ausgewählt ist, den Anteil an B-Zellen anzeigt, der Methylierungsgrad mindestens eines CpG-Dinukleotids, das aus der Gruppe bestehend aus den Nukleotiden Nr. 25 und 26, 61 und 62, 64 und 65, 81 und 82, und 104 und 105 der SEQ ID NR: 8 oder aus der Gruppe bestehend aus den Nukleotiden Nr. 22 und 23, 58 und 59, 61 und 62, 77 und 78, und 100 und 101 der SEQ ID NR: 9 oder aus der Gruppe bestehend aus den Nukleotiden Nr. 6 und 7, 42 und 43, 45 und 46, 61 und 62, 84 und 85, und 115 und 116 der SEQ ID NR: 10 ausgewählt ist, den Anteil an NK-Zellen anzeigt, der Methylierungsgrad mindestens eines CpG-Dinukleotids, das aus der Gruppe bestehend aus den Nukleotiden Nr. 17 und 18, 26 und 27, 34 und 35, 46 und 47, 61 und 62, 92 und 93, 96 und 97, und 108 und 109 der SEQ ID NR: 11 ausgewählt ist, den Anteil an T-Zellen anzeigt, der Methylierungsgrad der Nukleotide Nr. 20 und 21, und/oder 61 und 62 der SEQ ID NR: 12 den Anteil an CD4+ - T-Zellen anzeigt, und der Methylierungsgrad der Nukleotide Nr. 61 und 62 der SEQ ID NR: 13 den Anteil an CD8+ - T-Zellen anzeigt.

**2.** Verwendung eines künstlichen Nukleinsäuremoleküls zur Bestimmung zumindest eines Teils der zellulären Zusammensetzung von Blut nach dem Verfahren gemäß Anspruch 1, bei der das Nukleinsäuremolekül mindestens eine Nukleotidsequenz umfasst, die ausgewählt ist aus der Gruppe bestehend aus:

a) Einer Nukleotidsequenz, die aus der Gruppe bestehend aus der SEQ ID NR: 1, SEQ ID NR: 2, SEQ ID NR: 3, SEQ ID NR: 4, SEQ ID NR: 5, SEQ ID NR: 6, SEQ ID NR: 7, SEQ ID NR: 8, SEQ ID NR: 9, SEQ ID NR: 10, SEQ ID NR: 11, SEQ ID NR: 12, und SEQ ID NR: 13 ausgewählt ist;
b) einer Nukleotidsequenz, die sich von der Nukleotidsequenz gemäß a) durch den Austausch von höchstens 10 % der Nukleotide, vorzugsweise höchstens 5 % der Nukleotide, außer des/der CpG-Dinukleotid(s/e), unterscheidet; und
c) einer Nukleotidsequenz, die dem komplementären Strang der Nukleotidsequenz gemäß a) oder b) entspricht.

## Revendications

**1.** Procédé de détermination d'au moins une partie de la composition cellulaire du sang, comprenant :

- l'isolement d'ADN génomique d'au moins un échantillon de sang pour obtenir au moins une préparation d'acide nucléique dudit échantillon de sang ;
- l'identification des niveaux de méthylation de l'ADN d'au moins deux régions spécifiques différentes de ladite préparation d'acide nucléique, chaque région spécifique comprenant au moins un dinucléotide CpG, dans lequel chacune desdites deux régions spécifiques différentes comprend au moins une séquence nucléotidique sélectionnée dans le groupe consistant en SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, et SEQ ID NO: 13; et
- la détermination des proportions d'au moins deux types différents de cellules sanguines sur la base des niveaux de méthylation identifiés pour prédire la distribution relative desdits au moins deux types différents de cellules sanguines dans l'échantillon de sang, dans lequel le niveau de méthylation d'au moins un dinucléotide CpG sélectionné dans le groupe consistant en les nucléotides n° 6 et 7, 38 et 39, 49 et 50, 51 et 52, 61 et 62, 80 et 81, 89 et 90, et 112 et 113 de SEQ ID NO : 1 indique le pourcentage de granulocytes, le niveau de méthylation d'au moins un dinucléotide CpG sélectionné dans le groupe consistant en les nucléotides n° 40 et 41, 45 et 46, 53 et 54, et 61 et 62 de SEQ ID NO : 5 indique le pourcentage de monocytes, le niveau de méthylation des nucléotides n° 61 et 62 de SEQ ID NO : 6 indique le pourcentage de lymphocytes, le niveau de méthylation d'au moins un dinucléotide CpG sélectionné dans le groupe consistant en les nucléotides n° 15 et 16, 26 et 27, 32 et 33, et 61 et 62 de SEQ ID NO : 2 indique le pourcentage de granulocytes neutrophiles, le niveau de méthylation d'au moins un dinucléotide CpG sélectionné dans le groupe consistant en les nucléotides n° 6 et 7, 21 et 22, 32 et 33, 34 et 35, 54 et 55, 61 et 62, 63 et 64, 81 et 82, 93 et 94, 101 et 102, et 121 et 122 de SEQ ID NO : 3, ou sélectionné dans le groupe consistant en les nucléotides n° 1 et 2, 12 et 13, 14 et 15, 34 et 35, 41 et 42, 43 et 44, 61 et 62, 73 et 74, 81 et 82, et 101 et 102 de SEQ ID NO : 4 indique le pourcentage de granulocytes éosinophiles, le niveau de méthylation d'au moins un dinucléotide CpG sélectionné dans le groupe consistant en les nucléotides n° 5 et 6, 36 et 37, 42 et 43, 53 et 54, 58 et 59, 61 et 62, et 63 et 64 de SEQ ID NO : 7 indique le pourcentage de lymphocytes B, le niveau de méthylation d'au moins un dinucléotide CpG sélectionné dans le groupe consistant en les nucléotides n° 25 et 26, 61 et 62, 64 et 65, 81 et 82, et 104 et 105 de SEQ ID NO : 8, ou sélectionné dans le groupe consistant en les nucléotides n° 22 et 23, 58 et 59, 61 et 62, 77 et 78, et 100 et 101 de SEQ ID NO : 9, ou sélectionné dans le groupe consistant en les nucléotides n° 6 et 7, 42 et 43, 45 et 46, 61 et 62, 84 et 85, et 115 et 116 de SEQ ID NO : 10 indique le pourcentage de cellules NK, le niveau de méthylation d'au moins un dinucléotide CpG sélectionné dans le groupe consistant en les nucléotides n° 17 et 18, 26 et 27, 34 et 35, 46 et 47, 61 et 62, 92 et 93, 96 et 97, et 108 et 109 de SEQ ID NO: 11 indique le pourcentage de lymphocytes T, le niveau de méthylation des nucléotides n° 20 et 21, et /ou 61 et 62 de SEQ ID NO : 12 indique le pourcentage de lymphocytes T CD4+, et le niveau de méthylation des nucléotides n° 61 et 62 de SEQ ID NO : 13 indique le pourcentage de lymphocytes T CD8+.

**2.** Utilisation d'une molécule d'acide nucléique artificiel pour déterminer au moins une partie d'une composition cellulaire du sang conformément au procédé selon la revendication 1, dans laquelle la molécule d'acide nucléique comprend au moins une séquence nucléotidique sélectionnée dans le groupe consistant en :

a) une séquence nucléotidique sélectionnée dans le groupe consistant en SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, et SEQ ID NO : 13 ;
b) une séquence nucléotidique qui diffère de la séquence nucléotidique de a) par remplacement d'au plus 10 % des nucléotides, de préférence d'au plus 5 % des nucléotides, à l'exception du (des) dinucléotide(s) CpG ; et
c) une séquence nucléotidique qui correspond au brin complémentaire de la séquence nucléotidique de a) ou b).

Fig. 1

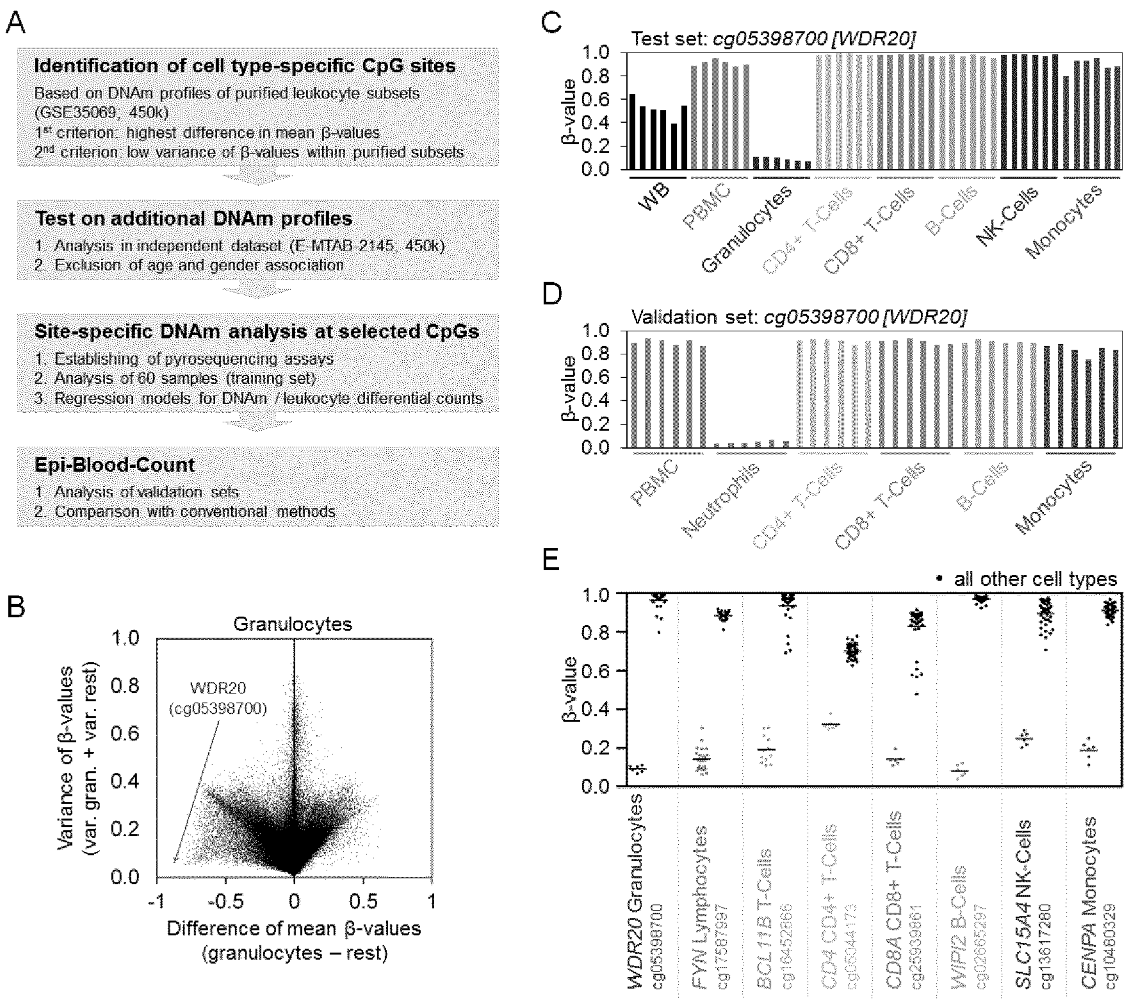
A
Identification of cell type-specific CpG sites
Based on DNAm profiles of purified leukocyte subsets (GSE35069; 450k)
1st criterion: highest difference in mean β-values
2nd criterion: low variance of β-values within purified subsets
Test on additional DNAm profiles
1. Analysis in independent dataset (E-MTAB-2145; 450k)
2. Exclusion of age and gender association
Site-specific DNAm analysis at selected CpGs
1. Establishing of pyrosequencing assays
2. Analysis of 60 samples (training set)
3. Regression models for DNAm / leukocyte differential counts
Epi-Blood-Count
1. Analysis of validation sets
2. Comparison with conventional methods
B
Granulocytes
WDR20 (cg05398700)
Variance of β-values (var. gran. + var. rest)
Difference of mean β-values (granulocytes – rest)
C
Test set: cg05398700 [WDR20]
β-value
WB
PBMC
Granulocytes
CD4+ T-Cells
CD8+ T-Cells
B-Cells
NK-Cells
Monocytes
D
Validation set: cg05398700 [WDR20]
β-value
PBMC
Neutrophils
CD4+ T-Cells
CD8+ T-Cells
B-Cells
Monocytes
E
• all other cell types
β-value
WDR20 Granulocytes cg05398700
FYN Lymphocytes cg17587997
BCL11B T-Cells cg16452866
CD4 CD4+ T-Cells cg05044173
CD8A CD8+ T-Cells cg25939861
WIPI2 B-Cells cg02665297
SLC15A4 NK-Cells cg13617280
CENPA Monocytes cg10480329

## Fig. 2

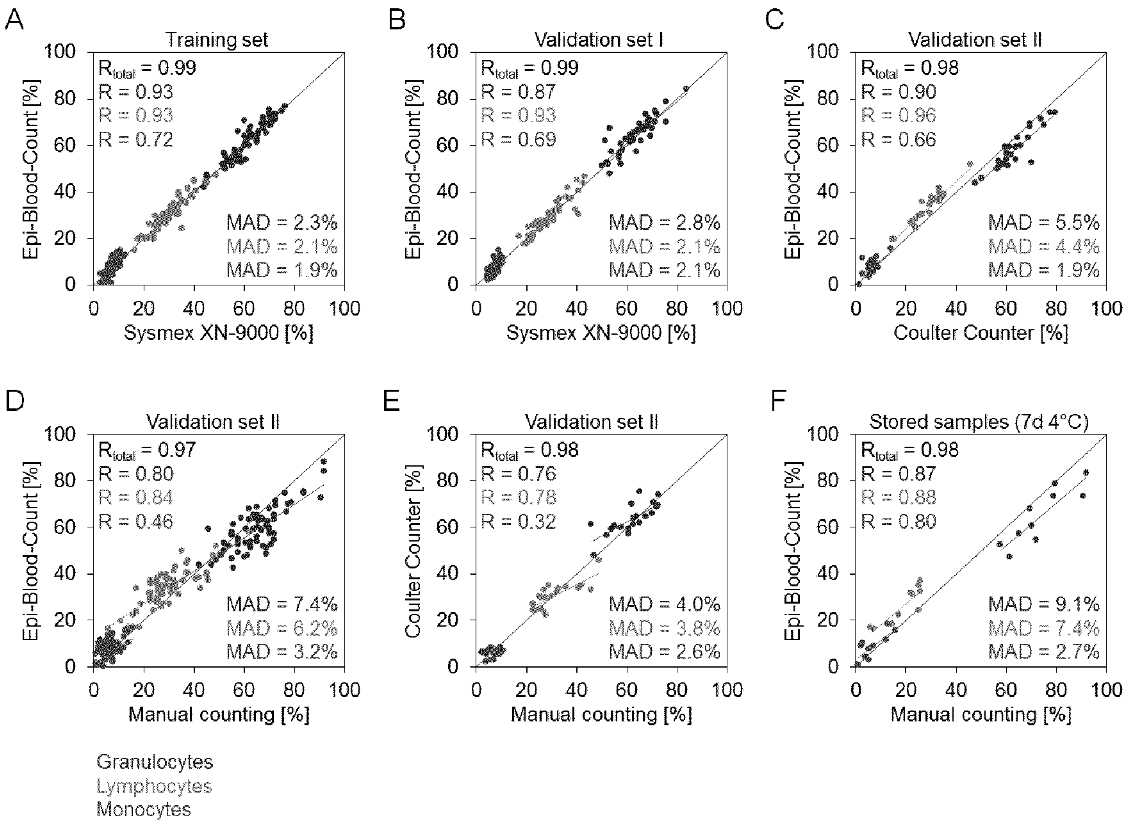
A
Training set
Rtotal = 0.99
R = 0.93
R = 0.93
R = 0.72
MAD = 2.3%
MAD = 2.1%
MAD = 1.9%
Epi-Blood-Count [%]
Sysmex XN-9000 [%]
B
Validation set I
Rtotal = 0.99
R = 0.87
R = 0.93
R = 0.69
MAD = 2.8%
MAD = 2.1%
MAD = 2.1%
Epi-Blood-Count [%]
Sysmex XN-9000 [%]
C
Validation set II
Rtotal = 0.98
R = 0.90
R = 0.96
R = 0.66
MAD = 5.5%
MAD = 4.4%
MAD = 1.9%
Epi-Blood-Count [%]
Coulter Counter [%]
D
Validation set II
Rtotal = 0.97
R = 0.80
R = 0.84
R = 0.46
MAD = 7.4%
MAD = 6.2%
MAD = 3.2%
Epi-Blood-Count [%]
Manual counting [%]
E
Validation set II
Rtotal = 0.98
R = 0.76
R = 0.78
R = 0.32
MAD = 4.0%
MAD = 3.8%
MAD = 2.6%
Coulter Counter [%]
Manual counting [%]
F
Stored samples (7d 4°C)
Rtotal = 0.98
R = 0.87
R = 0.88
R = 0.80
MAD = 9.1%
MAD = 7.4%
MAD = 2.7%
Epi-Blood-Count [%]
Manual counting [%]
Granulocytes
Lymphocytes
Monocytes

## Fig. 3

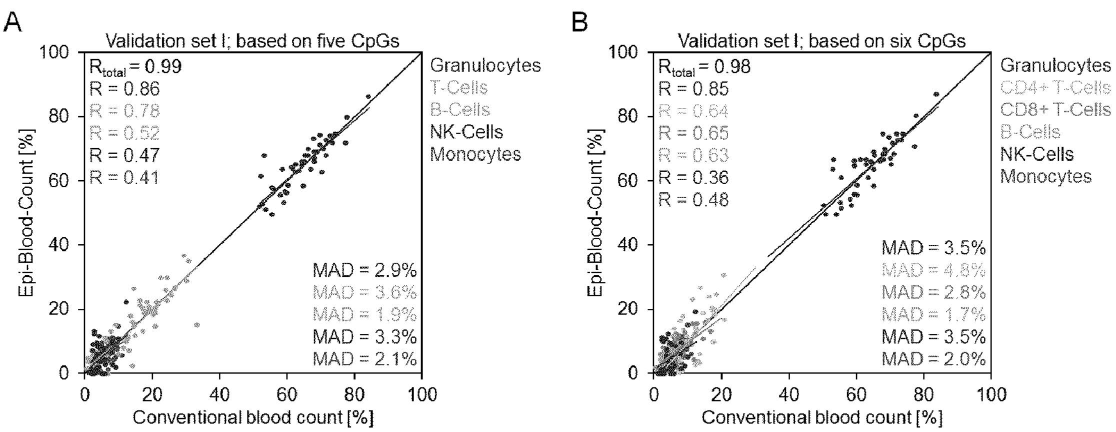
A
Validation set I; based on five CpGs
Rtotal = 0.99
R = 0.86
R = 0.78
R = 0.52
R = 0.47
R = 0.41
MAD = 2.9%
MAD = 3.6%
MAD = 1.9%
MAD = 3.3%
MAD = 2.1%
Granulocytes
T-Cells
B-Cells
NK-Cells
Monocytes
Epi-Blood-Count [%]
Conventional blood count [%]
B
Validation set I; based on six CpGs
Rtotal = 0.98
R = 0.85
R = 0.64
R = 0.65
R = 0.63
R = 0.36
R = 0.48
MAD = 3.5%
MAD = 4.8%
MAD = 2.8%
MAD = 1.7%
MAD = 3.5%
MAD = 2.0%
Granulocytes
CD4+ T-Cells
CD8+ T-Cells
B-Cells
NK-Cells
Monocytes
Epi-Blood-Count [%]
Conventional blood count [%]

Fig. 4

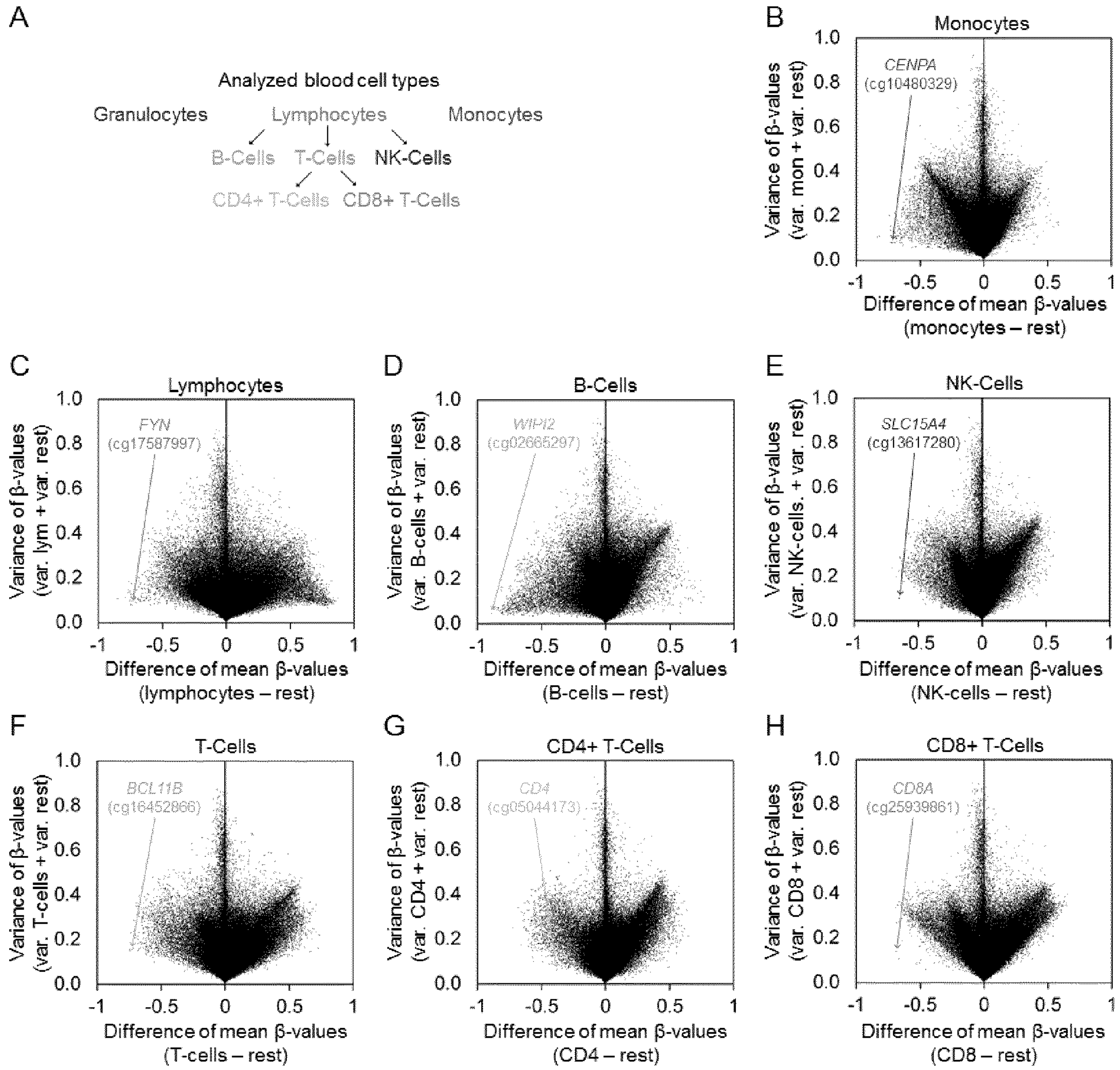
A
Analyzed blood cell types
Granulocytes
Lymphocytes
Monocytes
B-Cells
T-Cells
NK-Cells
CD4+ T-Cells
CD8+ T-Cells
B
Monocytes
CENPA
(cg10480329)
Variance of β-values (var. mon + var. rest)
Difference of mean β-values (monocytes – rest)
C
Lymphocytes
FYN
(cg17587997)
Variance of β-values (var. lym + var. rest)
Difference of mean β-values (lymphocytes – rest)
D
B-Cells
WIPI2
(cg02665297)
Variance of β-values (var. B-cells + var. rest)
Difference of mean β-values (B-cells – rest)
E
NK-Cells
SLC15A4
(cg13617280)
Variance of β-values (var. NK-cells. + var. rest)
Difference of mean β-values (NK-cells – rest)
F
T-Cells
BCL11B
(cg16452866)
Variance of β-values (var. T-cells + var. rest)
Difference of mean β-values (T-cells – rest)
G
CD4+ T-Cells
CD4
(cg05044173)
Variance of β-values (var. CD4 + var. rest)
Difference of mean β-values (CD4 – rest)
H
CD8+ T-Cells
CD8A
(cg25939861)
Variance of β-values (var. CD8 + var. rest)
Difference of mean β-values (CD8 – rest)

## Fig. 5

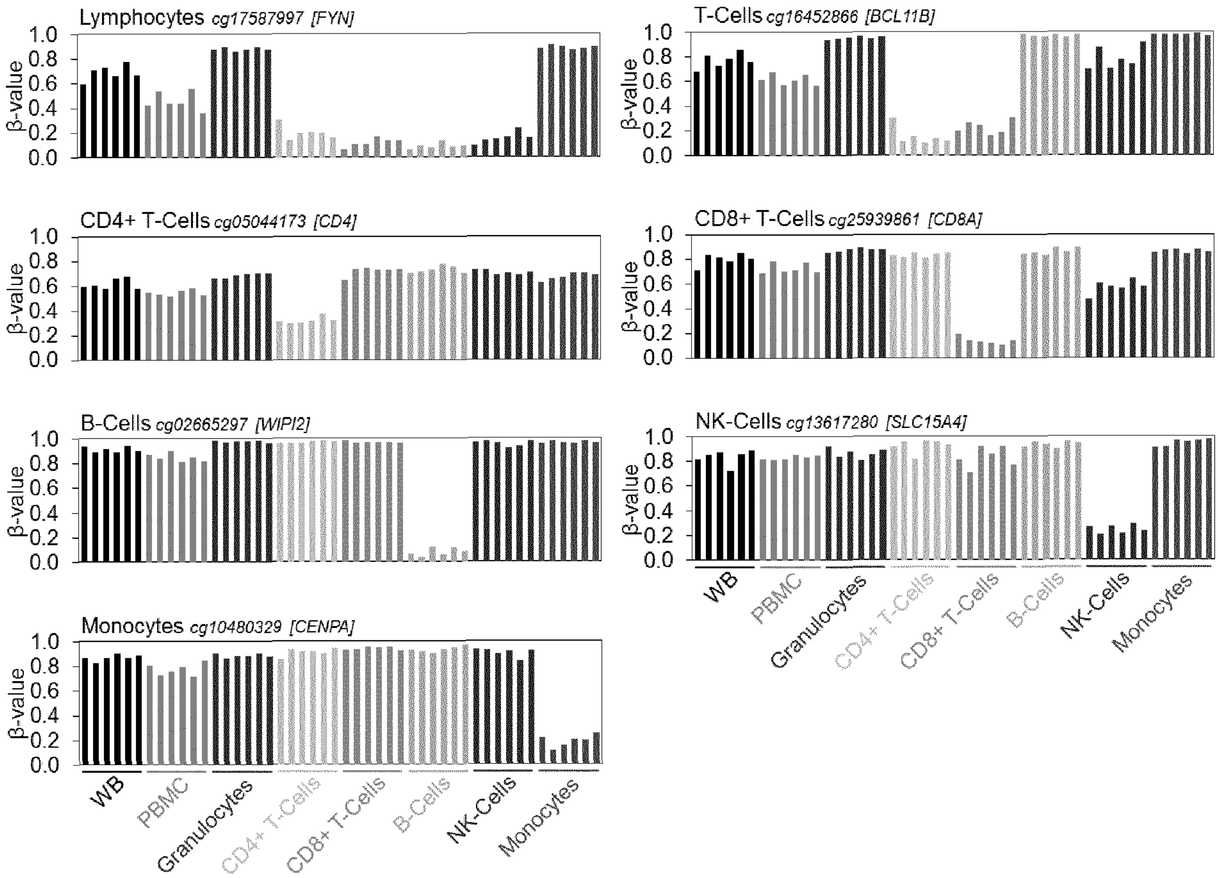
Lymphocytes cg17587997 [FYN]
T-Cells cg16452866 [BCL11B]
CD4+ T-Cells cg05044173 [CD4]
CD8+ T-Cells cg25939861 [CD8A]
B-Cells cg02665297 [WIPI2]
NK-Cells cg13617280 [SLC15A4]
Monocytes cg10480329 [CENPA]
β-value
1.0
0.8
0.6
0.4
0.2
0.0
WB
PBMC
Granulocytes
CD4+ T-Cells
CD8+ T-Cells
B-Cells
NK-Cells
Monocytes

**Fig. 6**

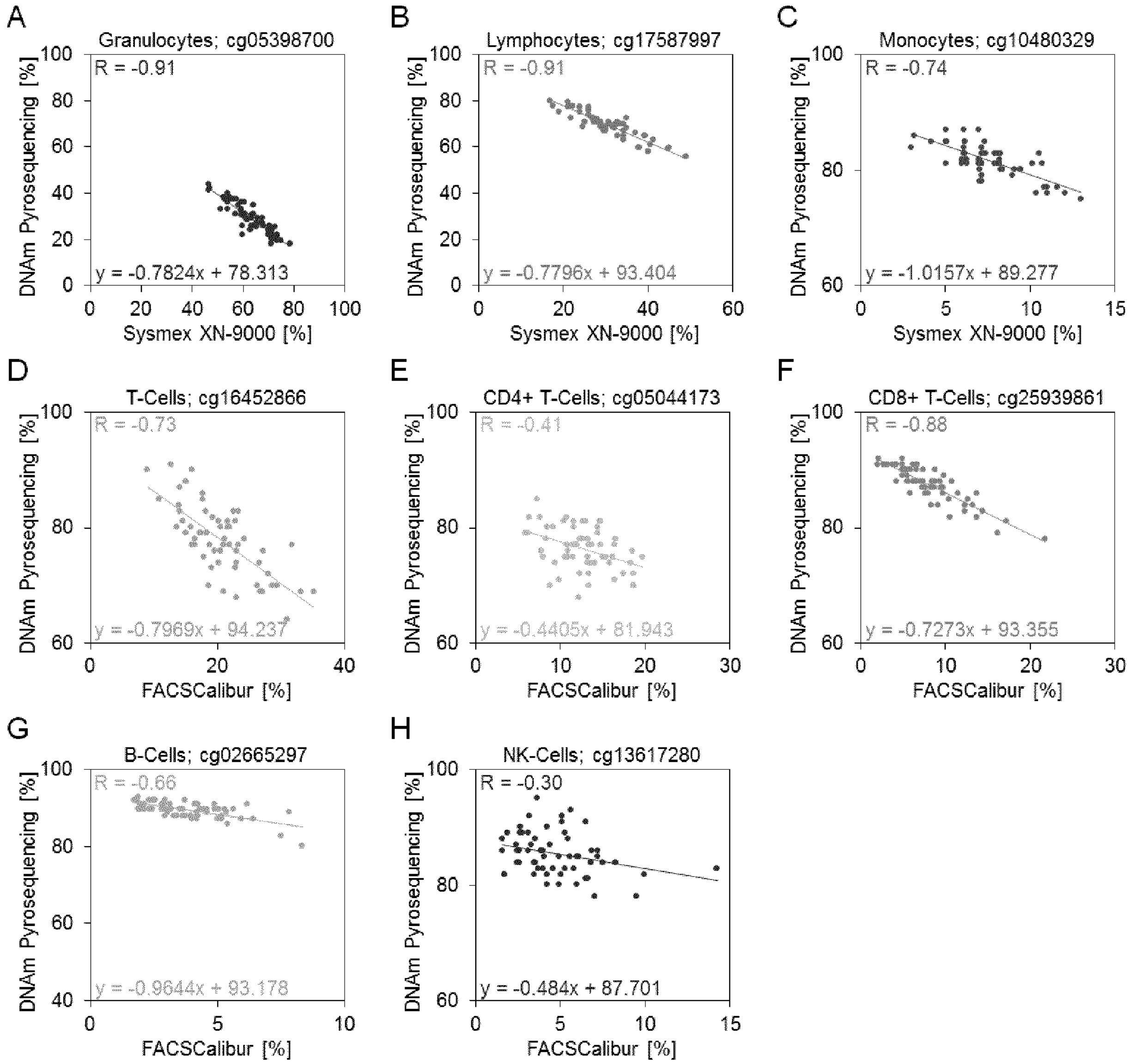
A
Granulocytes; cg05398700
R = -0.91
y = -0.7824x + 78.313
DNAm Pyrosequencing [%]
Sysmex XN-9000 [%]
B
Lymphocytes; cg17587997
R = -0.91
y = -0.7796x + 93.404
DNAm Pyrosequencing [%]
Sysmex XN-9000 [%]
C
Monocytes; cg10480329
R = -0.74
y = -1.0157x + 89.277
DNAm Pyrosequencing [%]
Sysmex XN-9000 [%]
D
T-Cells; cg16452866
R = -0.73
y = -0.7969x + 94.237
DNAm Pyrosequencing [%]
FACSCalibur [%]
E
CD4+ T-Cells; cg05044173
R = -0.41
y = -0.4405x + 81.943
DNAm Pyrosequencing [%]
FACSCalibur [%]
F
CD8+ T-Cells; cg25939861
R = -0.88
y = -0.7273x + 93.355
DNAm Pyrosequencing [%]
FACSCalibur [%]
G
B-Cells; cg02665297
R = -0.66
y = -0.9644x + 93.178
DNAm Pyrosequencing [%]
FACSCalibur [%]
H
NK-Cells; cg13617280
R = -0.30
y = -0.484x + 87.701
DNAm Pyrosequencing [%]
FACSCalibur [%]

## Fig. 7

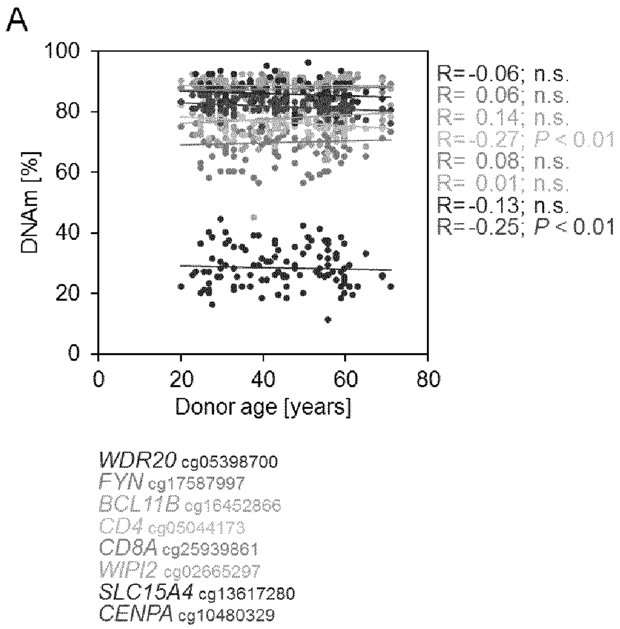
A
100
80
60
40
20
0
DNAm [%]
0
20
40
60
80
Donor age [years]
R=-0.06; n.s.
R= 0.06; n.s.
R= 0.14; n.s.
R=-0.27; P < 0.01
R= 0.08; n.s.
R= 0.01; n.s.
R=-0.13; n.s.
R=-0.25; P < 0.01
WDR20 cg05398700
FYN cg17587997
BCL11B cg16452866
CD4 cg05044173
CD8A cg25939861
WIPI2 cg02665297
SLC15A4 cg13617280
CENPA cg10480329

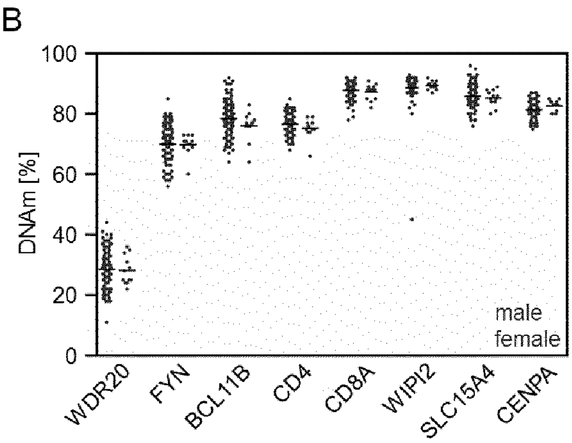
B
100
80
60
40
20
0
DNAm [%]
male
female
WDR20
FYN
BCL11B
CD4
CD8A
WIPI2
SLC15A4
CENPA

## Fig. 8

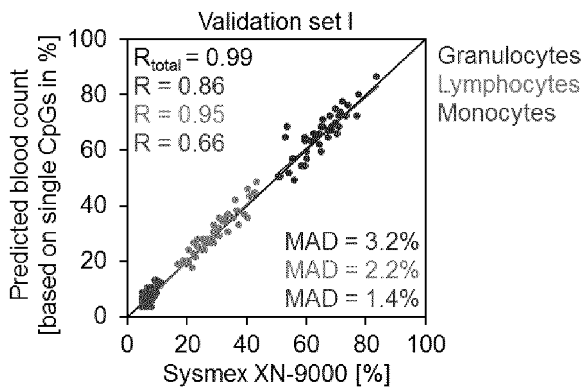
Validation set I
Predicted blood count
[based on single CpGs in %]
100
80
60
40
20
0
R_total = 0.99
R = 0.86
R = 0.95
R = 0.66
MAD = 3.2%
MAD = 2.2%
MAD = 1.4%
0
20
40
60
80
100
Sysmex XN-9000 [%]
Granulocytes
Lymphocytes
Monocytes

## Fig. 9

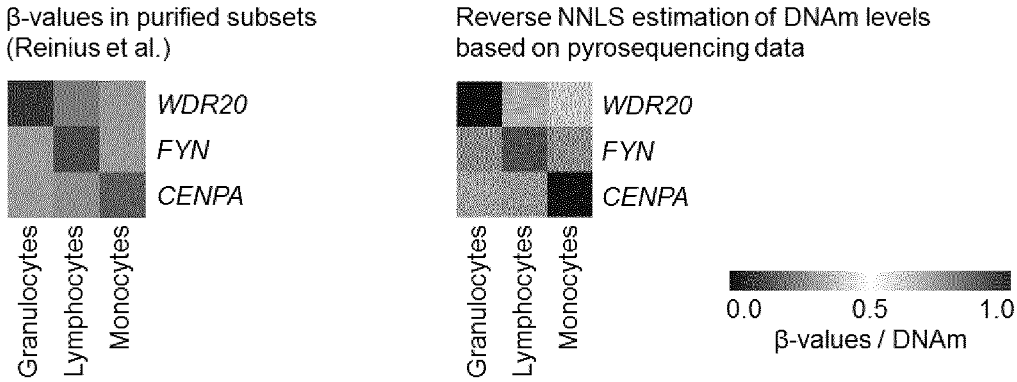


## Fig. 10

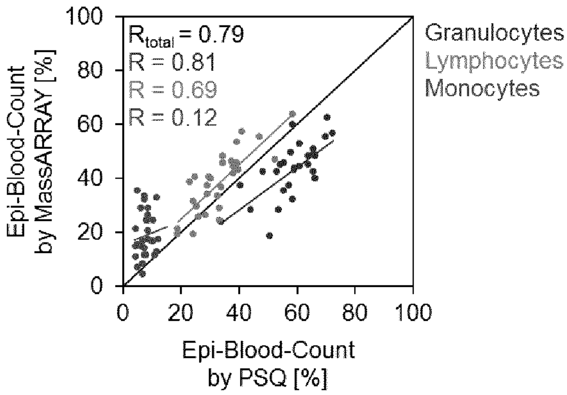


## Fig. 11

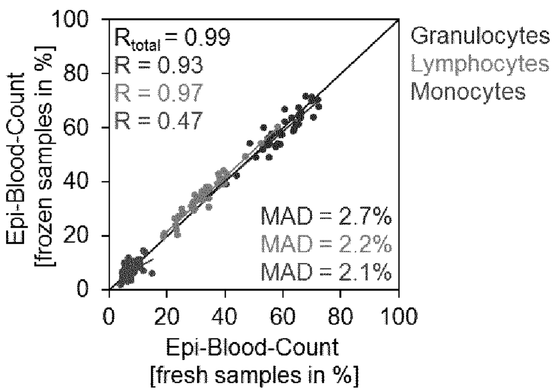

Fig. 12

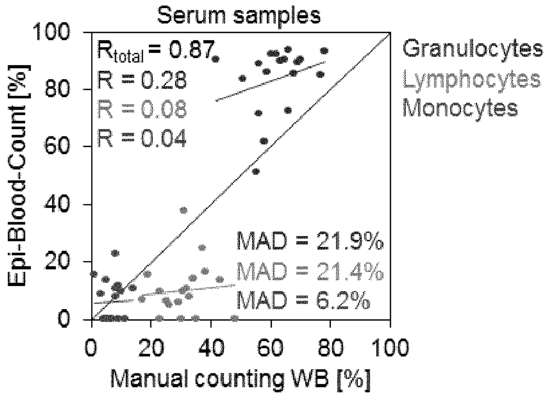
Serum samples
$R_{total}$ = 0.87
R = 0.28
R = 0.08
R = 0.04
Granulocytes
Lymphocytes
Monocytes
MAD = 21.9%
MAD = 21.4%
MAD = 6.2%
Epi-Blood-Count [%]
Manual counting WB [%]

Fig. 13

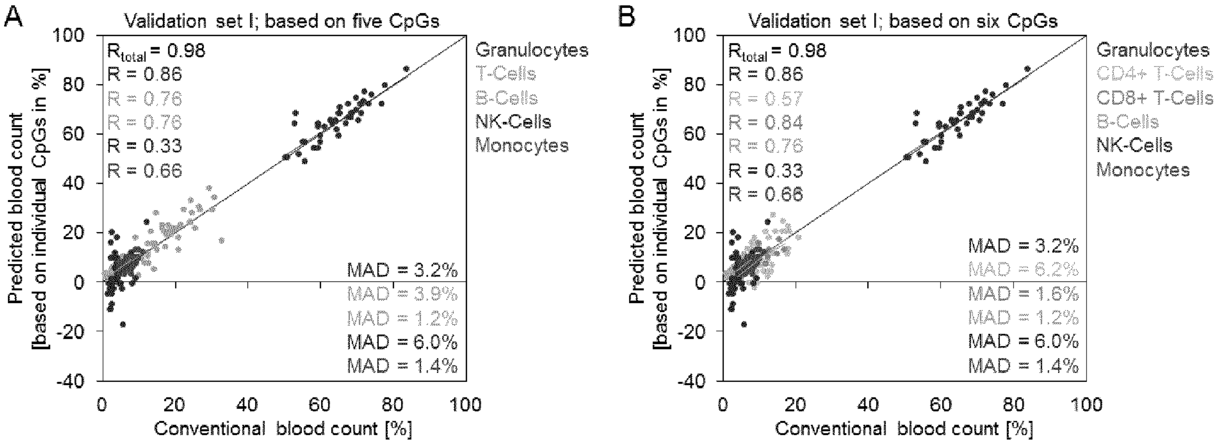
A
Validation set I; based on five CpGs
$R_{total}$ = 0.98
R = 0.86
R = 0.76
R = 0.76
R = 0.33
R = 0.66
Granulocytes
T-Cells
B-Cells
NK-Cells
Monocytes
MAD = 3.2%
MAD = 3.9%
MAD = 1.2%
MAD = 6.0%
MAD = 1.4%
Predicted blood count [based on individual CpGs in %]
Conventional blood count [%]
B
Validation set I; based on six CpGs
$R_{total}$ = 0.98
R = 0.86
R = 0.57
R = 0.84
R = 0.76
R = 0.33
R = 0.66
Granulocytes
CD4+ T-Cells
CD8+ T-Cells
B-Cells
NK-Cells
Monocytes
MAD = 3.2%
MAD = 6.2%
MAD = 1.6%
MAD = 1.2%
MAD = 6.0%
MAD = 1.4%
Predicted blood count [based on individual CpGs in %]
Conventional blood count [%]

# Fig. 14

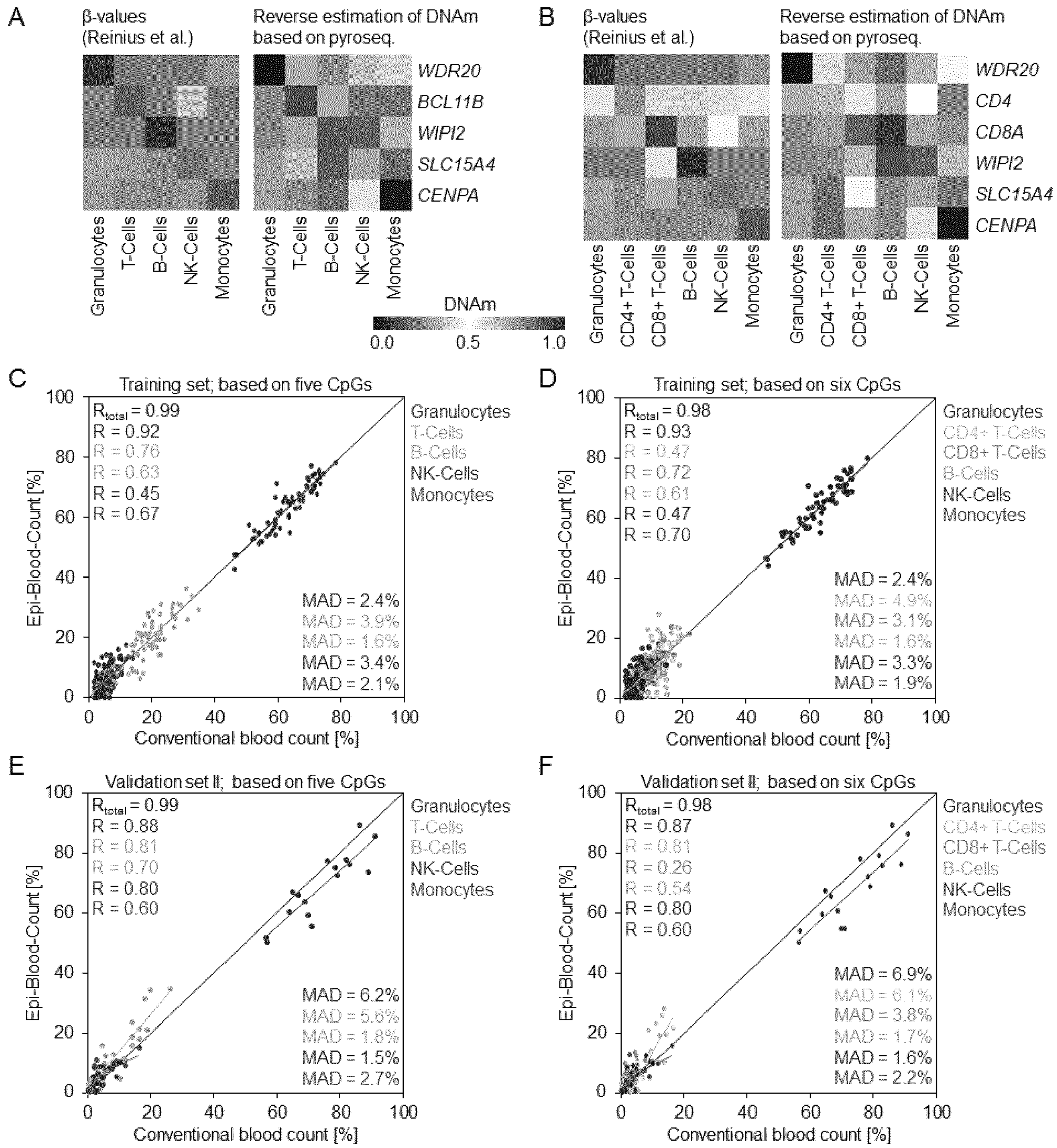
A
β-values (Reinius et al.)
Reverse estimation of DNAm based on pyroseq.
WDR20
BCL11B
WIPI2
SLC15A4
CENPA
Granulocytes
T-Cells
B-Cells
NK-Cells
Monocytes
DNAm
0.0
0.5
1.0
B
β-values (Reinius et al.)
Reverse estimation of DNAm based on pyroseq.
WDR20
CD4
CD8A
WIPI2
SLC15A4
CENPA
Granulocytes
CD4+ T-Cells
CD8+ T-Cells
B-Cells
NK-Cells
Monocytes
C
Training set; based on five CpGs
R_total = 0.99
R = 0.92
R = 0.76
R = 0.63
R = 0.45
R = 0.67
MAD = 2.4%
MAD = 3.9%
MAD = 1.6%
MAD = 3.4%
MAD = 2.1%
Granulocytes
T-Cells
B-Cells
NK-Cells
Monocytes
Epi-Blood-Count [%]
Conventional blood count [%]
D
Training set; based on six CpGs
R_total = 0.98
R = 0.93
R = 0.47
R = 0.72
R = 0.61
R = 0.47
R = 0.70
MAD = 2.4%
MAD = 4.9%
MAD = 3.1%
MAD = 1.6%
MAD = 3.3%
MAD = 1.9%
Granulocytes
CD4+ T-Cells
CD8+ T-Cells
B-Cells
NK-Cells
Monocytes
Epi-Blood-Count [%]
Conventional blood count [%]
E
Validation set II; based on five CpGs
R_total = 0.99
R = 0.88
R = 0.81
R = 0.70
R = 0.80
R = 0.60
MAD = 6.2%
MAD = 5.6%
MAD = 1.8%
MAD = 1.5%
MAD = 2.7%
Granulocytes
T-Cells
B-Cells
NK-Cells
Monocytes
Epi-Blood-Count [%]
Conventional blood count [%]
F
Validation set II; based on six CpGs
R_total = 0.98
R = 0.87
R = 0.81
R = 0.26
R = 0.54
R = 0.80
R = 0.60
MAD = 6.9%
MAD = 6.1%
MAD = 3.8%
MAD = 1.7%
MAD = 1.6%
MAD = 2.2%
Granulocytes
CD4+ T-Cells
CD8+ T-Cells
B-Cells
NK-Cells
Monocytes
Epi-Blood-Count [%]
Conventional blood count [%]

Fig. 15

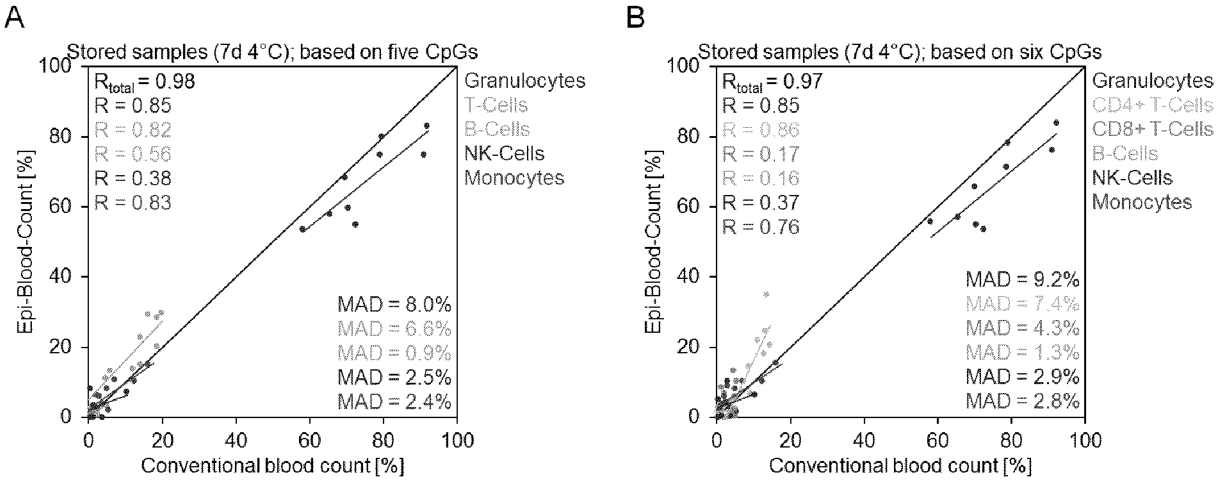
A
Stored samples (7d 4°C); based on five CpGs
$R_{total}$ = 0.98
R = 0.85
R = 0.82
R = 0.56
R = 0.38
R = 0.83
Granulocytes
T-Cells
B-Cells
NK-Cells
Monocytes
MAD = 8.0%
MAD = 6.6%
MAD = 0.9%
MAD = 2.5%
MAD = 2.4%
Epi-Blood-Count [%]
Conventional blood count [%]
0
20
40
60
80
100
B
Stored samples (7d 4°C); based on six CpGs
$R_{total}$ = 0.97
R = 0.85
R = 0.86
R = 0.17
R = 0.16
R = 0.37
R = 0.76
Granulocytes
CD4+ T-Cells
CD8+ T-Cells
B-Cells
NK-Cells
Monocytes
MAD = 9.2%
MAD = 7.4%
MAD = 4.3%
MAD = 1.3%
MAD = 2.9%
MAD = 2.8%
Epi-Blood-Count [%]
Conventional blood count [%]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014170497 A2
- EP 2199411 A1
- WO 2012162660 A2
- EP 2248913 A1
- US 20150004602 A1


### Non-patent literature cited in the description


- **WAITE et al.** *Frontiers in genetics,* 2016, vol. 7, 23
- **ABSHER, D.M. ; LI, X. ; WAITE, L.L. ; GIBSON, A. ; ROBERTS, K. ; EDBERG, J. ; CHATHAM, W.W. ; KIMBERLY, R.P.** Genome-wide DNA methylation analysis of systemic lupus erythematosus reveals persistent hypomethylation of interferon genes and compositional changes to CD4+ T-cell populations. *PLoS Genet,* 2013, vol. 9, e1003678
- **ACCOMANDO, W.P. ; WIENCKE, J.K. ; HOUSEMAN, E.A. ; NELSON, H.H. ; KELSEY, K.T.** Quantitative reconstruction of leukocyte subsets using DNA methylation. *Genome biology,* 2014, vol. 15, R50
- **ADALSTEINSSON, B.T. ; GUDNASON, H. ; ASPELUND, T. ; HARRIS, T.B. ; LAUNER, L.J. ; EIRIKSDOTTIR, G. ; SMITH, A.V. ; GUDNASON, V.** Heterogeneity in white blood cells has potential to confound DNA methylation measurements. *PloS one,* 2012, vol. 7, e46705
- **BLUEPRINT CONSORTIUM.** Quantitative comparison of DNA methylation assays for biomarker development and clinical applications. *Nature biotechnology,* 2016, vol. 34, 726-737
- **BUTTARELLO, M. ; PLEBANI, M.** Automated blood cell counts: state of the art. *Am J Clin Pathol,* 2008, vol. 130, 104-116
- **HAASE, H. ; FAHLENKAMP, A. ; SCHETTGEN, T. ; ESSER, A. ; GUBE, M. ; ZIEGLER, P. ; KRAUS, T. ; RINK, L.** Immunotoxicity monitoring in a population exposed to polychlorinated biphenyls. *International journal of environmental research and public health,* 2016, vol. 13
- **HOUSEMAN, E.A. ; ACCOMANDO, W.P. ; KOESTLER, D.C. ; CHRISTENSEN, B.C. ; MARSIT, C.J. ; NELSON, H.H. ; WIENCKE, J.K. ; KELSEY, K.T.** DNA methylation arrays as surrogate measures of cell mixture distribution. *BMC bioinformatics,* 2012, vol. 13, 86
- **HOUSEMAN, E.A. ; KIM, S. ; KELSEY, K.T. ; WIENCKE, J.K.** DNA Methylation in Whole Blood: Uses and Challenges. *Current environmental health reports,* 2015, vol. 2, 145-154
- **JAFFE, A.E. ; IRIZARRY, R.A.** Accounting for cellular heterogeneity is critical in epigenome-wide association studies. *Genome biology,* 2014, vol. 15, R31
- **KOESTLER, D.C. ; CHRISTENSEN, B. ; KARAGAS, M.R. ; MARSIT, C.J. ; LANGEVIN, S.M. ; KELSEY, K.T. ; WIENCKE, J.K. ; HOUSEMAN, E.A.** Blood-based profiles of DNA methylation predict the underlying distribution of cell types: a validation analysis. *Epigenetics,* 2013, vol. 8, 816-826
- **LEE, D.D. ; SEUNG, H.S.** Algorithms for non-negative matrix factorization. *Adv Neural Inform Process Systems,* 2001, vol. 13, 556-562
- **LIN, Q. ; WEIDNER, C.I. ; COSTA, I.G. ; MARIONI, R.E. ; FERREIRA, M.R. ; DEARY, I.J. ; WAGNER, W.** DNA methylation levels at individual age-associated CpG sites can be indicative for life expectancy. *Aging (Albany NY),* 2016, vol. 8, 394-401
- **MCGREGOR, K. ; BERNATSKY, S. ; COLMEGNA, I. ; HUDSON, M. ; PASTINEN, T. ; LABBE, A. ; GREENWOOD, C.M.** An evaluation of methods correcting for cell-type heterogeneity in DNA methylation studies. *Genome biology,* 2016, vol. 17, 84
- **REINIUS, L.E. ; ACEVEDO, N. ; JOERINK, M. ; PERSHAGEN, G. ; DAHLEN, S.E. ; GRECO, D. ; SODERHALL, C. ; SCHEYNIUS, A. ; KERE, J.** Differential DNA methylation in purified human blood cells: implications for cell lineage and studies on disease susceptibility. *PloS one,* 2012, vol. 7, e41361
- **SCHETTGEN, T. ; GUBE, M. ; ESSER, A. ; ALT, A. ; KRAUS, T.** Plasma polychlorinated biphenyls (PCB) levels of workers in a transformer recycling company, their family members, and employees of surrounding companies. *Journal of toxicology and environmental health,* 2012, vol. 75, 414-422

- **TESCHENDORFF, A.E. ; BREEZE, C.E. ; ZHENG, S.C. ; BECK, S.** A comparison of reference-based algorithms for correcting cell-type heterogeneity in Epigenome-Wide Association Studies. *BMC Bioinformatics,* 2017, vol. 18, 105
- **WAITE, L.L. ; WEAVER, B. ; DAY, K. ; LI, X. ; ROBERTS, K. ; GIBSON, A.W. ; EDBERG, J.C. ; KIMBERLY, R.P. ; ABSHER, D.M. ; TIWARI, H.K.** Estimation of Cell-Type Composition Including T and B Cell Subtypes for Whole Blood Methylation Microarray Data. *Frontiers in genetics,* 2016, vol. 7, 23
- **ZHENG, S.C. ; BECK, S. ; JAFFE, A.E. ; KOESTLER, D.C. ; HANSEN, K.D. ; HOUSEMAN, A.E. ; IRIZARRY, R.A. ; TESCHENDORFF, A.E.** Correcting for cell-type heterogeneity in epigenome-wide association studies: revisiting previous analyses. *Nat Methods,* 2017, vol. 14, 216-217
- **ZILBAUER, M. ; RAYNER, T.F. ; CLARK, C. ; COFFEY, A.J. ; JOYCE, C.J. ; PALTA, P. ; PALOTIE, A. ; LYONS, P.A. ; SMITH, K.G.** Genome-wide methylation analyses of primary human leukocyte subsets identifies functionally important cell-type-specific hypomethylated regions. *Blood,* 2013, vol. 122, e52-60

SEQUENCE LISTING

<110> RWTH Aachen

<120> Method for determining blood counts based on DNA methylation

<130> 16752EP

<160> 49

<170> PatentIn version 3.5

<210> 1
<211> 122
<212> DNA
<213> Homo sapiens

<400> 1

```
aggtgcggtg ggggaggggg attgggtcag aaaggcacgt gactcctccg cgctttgttt        60

cgtaaagcct gagactcccc gagtcttccg acttctccct gacatggccc tcgtttgggc       120

tt                                                                      122
```

<210> 2
<211> 122
<212> DNA
<213> Homo sapiens

<400> 2

```
gctgcctctg cttccgtttc ctccacgagg gcgaaggtat acccagaaca aattatgcca        60

cgaagatgca tgtctgacat ctttgttttt aaattgaatt cagttggtac tctggctttt       120

tt                                                                      122
```

<210> 3
<211> 122
<212> DNA

<213> Homo sapiens

<400> 3

gagcccgcaa cccctgacaa cgggaggaag acgcgccaca gggactggga cagcggccac 60

cgcggtgaca cctacagcca cgcaagcacc tgcgtaaaca cgtgctacag tcacatgtta 120

cg 122

<210> 4
<211> 122
<212> DNA
<213> Homo sapiens

<400> 4

cgggaggaag acgcgccaca gggactggga cagcggccac cgcggtgaca cctacagcca 60

cgcaagcacc tgcgtaaaca cgtgctacag tcacatgtta cgtatttcac acatttacac 120

ac 122

<210> 5
<211> 122
<212> DNA
<213> Homo sapiens

<400> 5

accttggcct cccagagtgc tgggattaca ggcctgagcc gctgcgcctg gccgtattta 60

cgatcttaag agaaagggca ggagtgtttc cttgacccca cattctcact tccctatacc 120

tc 122

<210> 6
<211> 122
<212> DNA
<213> Homo sapiens

<400> 6

taagtacttt gcataatgca aagcttgtgc ctctagctaa tttgcaggca gatgacacag 60

cgtgagaagc ctctgattta gtagaataag aactatttgt aaccaaacag cctccctctg 120

tt 122

<210> 7
<211> 122
<212> DNA
<213> Homo sapiens

<400> 7

ctgccgttgg gtttttttcat atctgtacat aatgccgagt gcgtaaggaa accgtggcgt 60

cgcgcacagt gggtctgctt gtcaaggcca gttctgcagt gacaggccca ggggctgccc 120

ac 122

<210> 8
<211> 122
<212> DNA
<213> Homo sapiens

<400> 8

catcaggagg cttggtgatg aaaacgctct ggccacagag gaagaccaca aaagcaaggc 60

cgacgcagac agtggggatc gcataaccag tgacaaagct gacgttctgc tgaatatagg 120

ca 122

<210> 9
<211> 122
<212> DNA
<213> Homo sapiens

<400> 9

caggaggctt ggtgatgaaa acgctctggc cacagaggaa gaccacaaaa gcaaggccga 60

cgcagacagt ggggatcgca taaccagtga caaagctgac gttctgctga atataggcaa 120

tg 122

<210> 10
<211> 122
<212> DNA
<213> Homo sapiens

<400> 10

gaaaacgctc tggccacaga ggaagaccac aaaagcaagg ccgacgcaga cagtggggat 60

cgcataacca gtgacaaagc tgacgttctg ctgaatatag gcaatgccac ctaacgacag 120

ga 122

<210> 11
<211> 122
<212> DNA
<213> Homo sapiens

<400> 11

cacagcctga caccaccgaa gtttccgggt gcccggatgc acagccgagc tgcctggtgc 60

cgctagaaca tcagaggact ctaagatgca ccgaacgcca ccatgggcgg ttatggaaga 120

ga 122

<210> 12
<211> 122
<212> DNA
<213> Homo sapiens

<400> 12

acagcctggt cagacatggc gctaccacta atggaatctt tcttgccatc tttttcttgc 60

cgcttaacag tggcagtgac agtttgactc ctgatttaag cctgattctg cttaactttt 120

tc 122

<210> 13
<211> 122
<212> DNA
<213> Homo sapiens

<400> 13

gatgatggcc agatttgggg gtgtactgct ttaaaataga ctagttccat cttagtctca 60

cggaaatcag cttgggggcc ttctagccct gcagctcaga aaagtgtcag ccagtggggt 120

gg 122

<210> 14
<211> 34
<212> DNA
<213> Artificial Sequence

<220>
<223> WDR20 pyrosequencing primer, forward

<400> 14
acgaagttta aacgagggtt atgttaggga gaag 34

<210> 15
<211> 31
<212> DNA
<213> Artificial Sequence

<220>
<223> WDR20 pyrosequencing primer, reverse

<400> 15
gccaacaaca ttacaaaaat acctcttacc a 31

<210> 16
<211> 18
<212> DNA
<213> Artificial Sequence

<220>
<223> WDR20 pyrosequencing primer, sequencing

<400> 16
agattcgggg agttttag 18

<210> 17
<211> 30
<212> DNA
<213> Artificial Sequence

<220>
<223> FYN pyrosequencing primer, forward

<400> 17
tagagggagg ttgtttggtt ataaatagtt 30

<210> 18
<211> 42
<212> DNA
<213> Artificial Sequence

<220>
<223> FYN pyrosequencing primer, reverse

<400> 18
cactttccaa catattaatt ataaaaataa atactttaca ta 42

<210> 19
<211> 22
<212> DNA
<213> Artificial Sequence

<220>
<223> FYN pyrosequencing primer, sequencing

<400> 19
ctaatttaca aacaaataac ac 22

<210> 20
<211> 35
<212> DNA
<213> Artificial Sequence

<220>
<223> PCYOX1 pyrosequencing primer, forward

<400> 20
tttgtaaaat aggattttta taggaatgga agtgt 35

<210> 21
<211> 34
<212> DNA
<213> Artificial Sequence

<220>
<223> PCYOX1 pyrosequencing primer, reverse

<400> 21
tcaaaaaaaa aaaaccaaaa taccaactaa attc 34

<210> 22
<211> 22
<212> DNA
<213> Artificial Sequence

<220>
<223> PCYOX1 pyrosequencing primer, sequencing

<400> 22
aaggtatatt tagaataaat ta 22

<210> 23
<211> 38
<212> DNA
<213> Artificial Sequence

<220>
<223> RPS6KA2 pyrosequencing primer, forward

<400> 23
gtagttatga gttcgtaatt tttgataacg ggaggaag 38

<210> 24
<211> 37
<212> DNA
<213> Artificial Sequence

<220>
<223> RPS6KA2 pyrosequencing primer, reverse

<400> 24
cgtcaaaact accaaaatac tttatcgttc ctacata 37

<210> 25
<211> 19
<212> DNA
<213> Artificial Sequence

<220>
<223> RPS6KA2 pyrosequencing primer, sequencing

<400> 25
gttataggga ttgggatag 19

<210> 26
<211> 33
<212> DNA
<213> Artificial Sequence

<220>
<223> BCL11B pyrosequencing primer, forward

<400> 26
gggattttgg gtcgacgtag atggtaaata aag 33

<210> 27
<211> 38
<212> DNA
<213> Artificial Sequence

<220>
<223> BCL11B pyrosequencing primer, reverse

<400> 27
cccataataa cgttcgatac atcttaaaat cctctaat 38

<210> 28
<211> 26
<212> DNA
<213> Artificial Sequence

<220>
<223> BCL11B pyrosequencing primer, sequencing

<400> 28
gggtgttcgg atgtatagtc gagttg 26

<210> 29
<211> 36
<212> DNA
<213> Artificial Sequence

<220>
<223> CD4 pyrosequencing primer, forward

<400> 29
ggaatatgtt aaagtgaaaa tgttaaagtt aaggga 36

<210> 30
<211> 27
<212> DNA
<213> Artificial Sequence

<220>
<223> CD4 pyrosequencing primer, reverse

<400> 30
catatacccc caactacctc ccacttc 27

<210> 31
<211> 23
<212> DNA
<213> Artificial Sequence

<220>
<223> CD4 pyrosequencing primer, sequencing

<400> 31
aataaaatct ttcttaccat ctt 23

<210> 32
<211> 31
<212> DNA

<213> Artificial Sequence

<220>
<223> CD8A pyrosequencing primer, forward

<400> 32
tttagtggtg gaagtattga gggggttaga g 31

<210> 33
<211> 37
<212> DNA
<213> Artificial Sequence

<220>
<223> CD8A pyrosequencing primer, reverse

<400> 33
cacttttcta aactacaaaa ctaaaaaacc cccaaac 37

<210> 34
<211> 17
<212> DNA
<213> Artificial Sequence

<220>
<223> CD8A pyrosequencing primer, sequencing

<400> 34
ctaaaaaacc cccaaac 17

<210> 35
<211> 24
<212> DNA
<213> Artificial Sequence

<220>
<223> WIPI2 pyrosequencing primer, forward

<400> 35
ttggtgggta gtttttgggt ttgt 24

<210> 36
<211> 32
<212> DNA
<213> Artificial Sequence

<220>
<223> WIPI2 pyrosequencing primer, reverse

<400> 36
ccacctaccg ttaaattttt tcatatctat ac 32

<210> 37
<211> 22
<212> DNA
<213> Artificial Sequence

<220>

<223> WIPI2 pyrosequencing primer, sequencing

<400> 37
aattggtttt gataagtaga tt 22

<210> 38
<211> 35
<212> DNA
<213> Artificial Sequence

<220>
<223> SLC15A4 pyrosequencing primer, forward

<400> 38
atgaaaacgt tttggttata gaggaagatt ataaa 35

<210> 39
<211> 35
<212> DNA
<213> Artificial Sequence

<220>
<223> SLC15A4 pyrosequencing primer, reverse

<400> 39
tcctatcgtt aaataacatt acctatattc aacaa 35

<210> 40
<211> 24
<212> DNA
<213> Artificial Sequence

<220>
<223> SLC15A4 pyrosequencing primer, sequencing

<400> 40
atagaggaag attataaaag taag 24

<210> 41
<211> 35
<212> DNA
<213> Artificial Sequence

<220>
<223> CENPA pyrosequencing primer, forward

<400> 41
attatgttgg ttaggttggt ttttaattgt taatt 35

<210> 42
<211> 33
<212> DNA
<213> Artificial Sequence

<220>
<223> CENPA pyrosequencing primer, reverse

<400> 42

aaaatcaaaa aaacactcct accctttctc tta 33

<210> 43
<211> 20
<212> DNA
<213> Artificial Sequence

<220>
<223> CENPA pyrosequencing primer, sequencing

<400> 43
aaacactcct accctttctc 20

<210> 44
<211> 33
<212> DNA
<213> Artificial Sequence

<220>
<223> WDR20 MassArray primer, forward

<400> 44
aggaagagag gttagggtgg gagttattga gga 33

<210> 45
<211> 57
<212> DNA
<213> Artificial Sequence

<220>
<223> WDR20 MassArray primer, reverse

<400> 45
cagtaatacg actcactata gggagaaggc tccaacaaca ttacaaaaat acctctt 57

<210> 46
<211> 36
<212> DNA
<213> Artificial Sequence

<220>
<223> FYN MassArray primer, forward

<400> 46
aggaagagag gtgatttggt ttatttttga gtgaga 36

<210> 47
<211> 57
<212> DNA
<213> Artificial Sequence

<220>
<223> FYN MassArray primer, reverse

<400> 47
cagtaatacg actcactata gggagaaggc tttctaaaaa cactaaaaaa acccctt 57

<210> 48

<211> 36
<212> DNA
<213> Artificial Sequence

<220>
<223> CENPA MassArray primer, forward

<400> 48
aggaagagag ggggttaagg aaatattttt gttttt 36

<210> 49
<211> 56
<212> DNA
<213> Artificial Sequence

<220>
<223> CENPA MassArray primer, reverse

<400> 49
cagtaatacg actcactata gggagaaggc ttcaaactaa tctccaacta ccaacc 56